# EUROPÄISCHE PATENTSCHRIFT

(11) **EP 2 358 337 B1**
(45) Veröffentlichungstag und Bekanntmachung des Hinweises auf die Patenterteilung: **04.11.2015**
(21) Anmeldenummer: 09760878.0
(22) Anmeldetag: 30.11.2009
(51) Int. Cl.: A61Q 5/08, A61K 8/34, A61K 8/42, A61K 8/49, A61K 8/44, A61K 8/46, A61Q 5/00, A61K 8/86

(54) **REDUKTIVE ENTFÄRBUNG KERATINHALTIGER FASERN**
REDUCTIVE DECOLORIZATION OF FIBRES CONTAINING KERATIN
DÉCOLORATION RÉDUCTRICE DE FIBRES KÉRATINIQUES

(30) Priorität: 16.12.2008 DE 102008062237
(43) Veröffentlichungstag der Anmeldung: 24.08.2011
(73) Patentinhaber: Henkel AG & Co. KGaA, 40589 Düsseldorf (DE)
(72) Erfinder: EMMERLING, Winfried, 25436 Tornesch (DE); LISSNER, Yvonne, 21073 Hamburg (DE)
(86) Internationale Anmeldenummer: PCT/EP2009/066047
(87) Internationale Veröffentlichungsnummer: WO 2010/072515

(56) Entgegenhaltungen:
- WO-A1-99/07339
- DE-A1- 19 810 688
- DE-A1-102004 045 353
- US-A- 4 859 459
- US-A- 5 451 405
- US-A1- 2008 085 249

## Beschreibung

Die Erfindung betrifft Mittel mit einem sauren pH-Wert, enthaltend eine Kombination aus (a) mindestens einer organischen Verbindung, die mindestens eine Thiolgruppe und mindestens eine gegebenenfalls derivatisierte Carboxylgruppe trägt, (b) mindestens einer organischen Verbindung, ausgewählt aus der Gruppe, die gebildet wird aus cyclischen, organischen Carbonaten und C₄-C₁₂-Fettsäuredimethylamiden, (c) mindestens einen (C₁₀ bis C₃₀)-Fettalkohol und (d) mindestens einen Emulgator ausgewählt aus der Gruppe, umfassend mit (C₁₀ bis C₃₀)-Alkylgruppen substituierte zwitterionische Emulgatoren. Gleichfalls sind die Verwendung dieser Mittel für die Entfärbung keratinhaltiger Fasern, beispielsweise menschlicher Haare, und ein Verfahren zur Entfärbung keratinhaltiger Fasern, Gegenstand der Erfindung.

Beim Färben wird der Farbstoff auf das Substrat durch Adsorption an die Oberfläche, durch Eindiffundieren, durch Bildung auf und/oder in dem Substrat bzw. durch chemische Bindung übertragen. Zuerst wurden natürliche Farbstoffe, wie z.B. Purpur oder Carmin, verwendet. Durch den rasanten Fortschritt der Wissenschaften wurden über die Jahre für jede Anwendung maßgeschneiderte, synthetische Farbstoffe zugänglich. Für das Färben, beispielsweise von Papier, Textilien oder keratinhaltigen Fasern, kommen im allgemeinen entweder direktziehende Farbstoffe oder Oxidationsfarbstoffe in Frage. Oxidationsfarbstoffe entstehen durch oxidative Kupplung einer oder mehrerer Entwicklerkomponenten untereinander oder mit einer oder mehreren Kupplerkomponenten. Kuppler- und Entwicklerkomponenten werden auch als Oxidationsfarbstoffvorprodukte bezeichnet. Die oxidative Kupplung findet bevorzugt während der Färbevorgangs statt, damit die Farbstoffvorprodukte in das Substrat hinein diffundieren können und der Farbstoff sich in dem Substrat bildet. Durch die Größe des entstandenen Farbstoffmoleküls wird ein Auswaschen aus dem Substrat erschwert.

Als Entwicklerkomponenten werden üblicherweise primäre aromatische Amine mit einer weiteren, in para- oder ortho-Position befindlichen freien oder substituierten Hydroxy- oder Aminogruppe, Diaminopyridinderivate, heterocyclische Hydrazone, 4-Aminopyrazolonderivate sowie 2,4,5,6-Tetraaminopyrimidin und dessen Derivate eingesetzt.

Spezielle Vertreter sind beispielsweise p-Phenylendiamin, p-Toluylendiamin, 2,4,5,6-Tetraaminopyrimidin, p-Aminophenol, N,N-Bis-(2-hydroxyethyl)-p-phenylendiamin, 2-(2,5-Diaminophenyl)-ethanol, 2-(2,5-Diaminophenoxy)-ethanol, 1-Phenyl-3-carboxyamido-4-amino-pyrazol-5-on, 4-Amino-3-methylphenol, 2-Aminomethyl-4-aminophenol, 2-Hydroxymethyl-4-aminophenol, 2-Hydroxy-4,5,6-triaminopyrimidin, 2,4-Dihydroxy-5,6-diaminopyrimidin, 2,5,6-Triamino-4-hydroxy-pyrimidin und 4,5-Diamino-1-(2-hydroxyethyl)pyrazol.

Als Kupplerkomponenten werden in der Regel m-Phenylendiaminderivate, Naphthole, Resorcin und Resorcinderivate, Pyrazolone, m-Aminophenole und substituierte Pyridinderivate verwendet. Als Kupplersubstanzen eignen sich insbesondere α-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 2,4-Diaminophenoxyethanol, 2-Amino-4-(2-hydroxyethylamino)-anisol (Lehmanns Blau), 1-Phenyl-3-methyl-pyrazol-5-on, 2,4-Dichlor-3-aminophenol, 1,3-Bis-(2,4-diaminophenoxy)-propan, 2-Chlorresorcin, 4-Chlorresorcin, 2-Chlor-6-methyl-3-aminophenol, 2-Methylresorcin, 5-Methylresorcin, 3-Amino-6-methoxy-2-methylamino-pyridin und 3,5-Diamino-2,6-dimethoxypyridin.

Unter direktziehenden Farbstoffen werden im allgemeinen Farbstoffe verstanden, die bereits vor Beginn des Färbens vorgebildet sind und auf das Substrat aufziehen. Wichtige Vertreter dieser Farbstoffklasse sind beispielsweise Triphenylmethanfarbstoffe, Azofarbstoffe, Anthrachinonfarbstoffe oder Nitrobenzolfarbstoffe, welche jeweils kationische oder anionische Gruppen tragen können.

Ein wichtiges technisches Gebiet stellt neben dem Färben das Abziehen von Farbstoffen dar. Darunter versteht man im Allgemeinen das Entfernen von Färbungen oder Bedruckungen durch Auswaschen, chemische Veränderung oder Zerstörung des Farbstoffes. Die oxidative oder die reduktive Entfärbung gefärbter Materialien findet insbesondere bei der Entfärbung von Textilien oder Haaren Anwendung.

Eine oxidative Entfärbung führt meist zu guten Ergebnissen. Jedoch kann durch die starke Oxidationswirkung des zur Entfärbung benutzten Oxidationsmittels die Struktur des Substrats chemisch verändert werden. Dies geht mit einer unerwünschten physikalischen Änderung des Substrats einher. Textilien oder Haare können beispielsweise spröde werden oder insbesondere bei mehrmaliger Entfärbung gar brechen. Der visuelle Eindruck, die Haptik aber auch die Haltbarkeit des Substrats werden dadurch negativ beeinflusst.

Weniger Einfluss auf die Substratstruktur, insbesondere auf die Struktur keratinhaltiger Fasern, nehmen reduktive Entfärbemittel. Die reduktiven Entfärbemittel entfärben kaum die Naturhaarfarbe, sondern wirken lediglich auf die durch synthetische Färbung aufgetragenen Farbstoffe reduktiv ein. Es tritt somit kaum eine Aufhellung des Haars auf.

Die Druckschrift EP-A1-943 316 betrifft die Verwendung von Thiolgruppenhaltigen Verbindungen in Kombination mit alpha-Ketocarbonsäuren in Mitteln zur Entfärbung gefärbten Haars.

Die Druckschrift DE-A-102004045353 hat die Entfärbung gefärbter Haare unter Zuhilfenahme von Glyzerin oder Derivaten davon, organischen Carbonaten oder von Fettsäuredimethylamiden zum Gegenstand.

Die Patentanmeldung DE 102007039954.7 betrifft Mittel zur Haarentfärbung, enthaltend eine Kombination aus (a) mindestens einer organischen Verbindung, die mindestens eine Thiolgruppe und mindestens eine gegebenenfalls derivatisierte Carboxylgruppe trägt, (b) mindestens einer organischen Verbindung, ausgewählt aus der Gruppe, die gebildet wird aus cyclischen, organischen Carbonaten, Glycerin und seinen Derivaten und C₄-C₁₂-Fettsäuredimethylamiden. Allerdings lassen sich diese Mittel nicht mir den üblichen Verdickungsmitteln wie Hydroxyethylcellulsoen oder Carboxymethylcellulosen bzw. den allseits bekannten acrylsäurebasierten verdickenden Polymeren stabil verdicken.

Folglich bedürfen die Entfärbemittel des Standes der Technik einer Verbesserung der Viskositätseinstellung und der Lagerstabilität, ohne an der Entfärbekraft und den weiteren Wirkparametern Einbußen zu erleiden.

Aufgabe der vorliegenden Erfindung ist es, reduktive Entfärbemittel bereitzustellen, die das Substrat dauerhaft ohne Nachdunkelung entfärben. Die Substratstruktur soll dabei geschont werden. Ferner sollten die in den Entfärbemitteln eingesetzten Reduktionsmittel für eine kosmetische Verwendung physiologisch verträglich und toxikologisch unbedenklich sein. Die Einwirkzeit der Entfärbemittel soll möglichst gering sein. Insbesondere soll das Entfärbemittel eine stabile Viskosität besitzen und lagerfähig sein.

Überraschenderweise eignen sich die erfindungsgemäßen Mittel bzw. die darin enthaltene Wirkstoffkombination hervorragend zur beschleunigten und verbesserten Entfärbung gefärbter Substrate, wie beispielsweise Papier, Textilien oder keratinhaltigen Fasern, insbesondere menschlichem Haar. Die erfindungsgemäße Wirkstoffkombination eignet sich besonders zur faserschonenden Entfärbung keratinhaltiger Fasern. Das Mittel zeigt eine verbesserte Handhabbarkeit. Die Viskositätseinstellung ist über einen langen Zeitraum selbst bei Temperaturen von 40°C stabil.

Unter keratinhaltigen Fasern sind beispielsweise Wolle, Pelze, Federn und insbesondere menschliche Haare zu verstehen.

Ein erster Gegenstand der Erfindung sind daher Mittel zur reduktiven Entfärbung keratinhaltiger Fasern, insbesondere menschlicher Haare, mit einem pH-Wert von 2 bis 6,5 enthaltend in einem Träger eine Wirkstoffkombination aus
(a) mindestens einer organischen Verbindung der Formel (I)

   HS-X-COOM (I)

   worin
   X ein gesättigtes oder ungesättigtes, lineares oder verzweigtes sowie aliphatisches Kohlenwasserstoffgerüst bedeutet, das gegebenenfalls mit mindestens einer der folgenden Gruppen substituiert ist
   - Thiolgruppe
   - Carboxylgruppe
   - Carboxylatgruppe
   - Hydroxygruppe
   - -NH₂
   - (C₁ bis C₆)-Alkylamino
   - (C₁ bis C₆)-Dialkylamino
   - (C₁ bis C₆)-Hydroxyalkyl
   M steht für ein Wasserstoffatom, eine (C₁ bis C₈)-Alkylgruppe oder ein Äquivalent eines ein- oder mehrwertigen Kations,
(b) mindestens einer organischen Verbindung, ausgewählt aus
   (i) cyclischen, organischen Carbonaten und
      C₄-C₁₂-Fettsäuredimethylamiden,
(c) mindestens einen (C₁₀ bis C₃₀)-Fettalkohol und
(d) mindestens einen Emulgator ausgewählt aus mit mindestens einem (C₁₀ bis C₃₀)-Kohlenwasserstoffrest substituierten amphoteren Emulgatoren.

Das erfindungsgemäße Mittel weist bevorzugt einen pH-Wert von pH 2,5 bis pH 5, insbesondere einen pH-Wert von pH 3 bis pH 4 auf.

Die erzielte Viskosität des erfindungsgemäßen Mittels wird selbst bei einer 6 monatigen Lagerung bei einer Temperatur von 40°C beibehalten. Das erfindungsgemäße Mittel ist bevorzugt besonders wirksam, wenn es eine Viskosität von 2000 bis 10000 mPa·s, insbesondere von 3000 bis 6000 mPa·s aufweist (jeweils Brookfield DV-II+, Spindel 4, 20 UpM bei 20°C).

Es ist erfindungsgemäß bevorzugt, wenn die Verbindungen der Komponente (c) und die Verbindungen der Komponente (d) in einem Gewichtsverhältnis von 5 zu 1 bis 20 zu 1, besonders bevorzugt von 8 zu 1 bis 12 zu 1, in dem erfindungsgemäßen Mittel enthalten sind.

Gemäß Formel (I) ist es bevorzugt, wenn X für Methylen, Ethan-1,1-diyl, Ethan-1,2-diyl, Propan-1,1-diyl, Propan-1,2-diyl, Propan-1,3-diyl oder Butan-1,4-diyl steht, wobei jede dieser Gruppen gegebenenfalls mit mindestens einer der folgenden Reste substituiert sein kann
- Thiolgruppe
- Carboxylgruppe
- Carboxylatgruppe
- Hydroxygruppe
- -NH₂
- (C₁ bis C₆)-Alkylaminogruppe
- (C₁ bis C₆)-Dialkylaminogruppe.

Besonders bevorzugt steht in Formel (I) X für eine Gruppe aus
- Methylen,
- Ethan-1,1-diyl
- Ethan-1,2-diyl oder
- Propan-1,1-diyl steht,
wobei jede dieser Gruppen gegebenenfalls mit mindestens einer der folgenden Reste substituiert sein kann
- Carboxylgruppe
- Carboxylatgruppe
- Hydroxygruppe
- -NH₂

Ganz besonders bevorzugt steht in Formel (I) X für eine Ethan-1,2-diyl-Gruppe wobei diese Gruppe mit mindestens einem der folgenden Reste substituiert ist
- Carboxylgruppe
- Carboxylatgruppe
- Hydroxygruppe
- -NH₂

Jeweils gemäß Formel (I) zitierte (C₁ bis C₈)-Alkylreste (auch in den (C₁ bis C₆)-Alkylamino- sowie (C₁ bis C₆)-Dialkylaminogruppen) stehen bevorzugt für (bzw. leiten sich bevorzugt ab von) Methyl, Ethyl, Propyl, Isopropyl, n-Butyl, sec-Butyl, iso-Butyl, tert.-Butyl, n-Pentyl, iso-Pentyl, sec-Pentyl, n-Hexyl, 2-Methylpentyl, n-Heptyl, n-Octyl, 6-Methylheptyl, 2-Ethylhexyl oder 1,1,3,3-Tetramethylbutyl.

Wenn die Verbindungen der Formel (I) als Salz vorliegen, steht M für ein Äquivalent eines einoder mehrwertigen Kations. Das ein oder mehrwertige Kation M^{z+} mit einer Ladungszahl z von eins oder höher dient lediglich aus Gründen der Elektroneutralität zur Kompensation der einfach negativen Ladung des bei Salzbildung vorliegenden Carboxylatfragments -COO- in Formel (I). Das dafür zu verwendende Äquivalent des entsprechenden Kations beträgt 1/z. Das Fragment - COOM der Formeln (I) steht im Fall der Salzbildung für die Gruppe:

-COO⁻ 1/z (M^{z+}).

Als ein- oder mehrwertiges Kation M^{z+} kommen prinzipiell alle physiologisch verträglichen Kationen in Frage. Insbesondere sind dies Metallkationen der physiologisch verträglichen Metalle aus den Gruppen la, Ib, lia, lib, IIIb, Via oder VIII des Periodensystems der Elemente, Ammoniumionen, sowie kationische organische Verbindungen mit quaterniertem Stickstoffatom. Letztere werden beispielsweise durch Protonierung primärer, sekundärer oder tertiärer organischer Amine mit einer Säure, wie z.B. mit Verbindungen der Formel (I) in ihrer sauren Form, oder durch permanente Quaternisierung besagter organischer Amine gebildet. Beispiele dieser kationischen organischen Ammoniumverbindungen sind 2-Ammonioethanol und 2-Trimethylammonioethanol. M steht in Formel (I) bevorzugt für ein Wasserstoffatom, ein Ammoniumion, ein Alkalimetallion, für ein halbes Äquivalent eines Erdalkalimetallions oder ein halbes Äquivalent eines Zinkions, besonders bevorzugt für ein Wasserstoffatom, ein Ammoniumion, ein Natriumion, ein Kaliumion, ½ Kalziumion, ½ Magnesiumion oder ½ Zinkion.

Besonders bevorzugt enthält das erfindungsgemäße Mittel als Komponente (a) mindestens eine Verbindung, ausgewählt aus mindestens einem Vertreter der Gruppe, die gebildet wird, aus L-Cystein (Säure oder Salz), D-Cystein (Säure oder Salz), D,L-Cystein (Säure oder Salz), und Acetylcystein. Ganz besonders bevorzugt geeignet ist jegliches Stereoisomer von Cystein jeweils als Säure oder Salz.

Die Komponente (a) ist in den erfindungsgemäßen Mitteln bevorzugt in einer Menge von 1 bis 10 Gew.-%, insbesondere von 1 bis 5 Gew.-%, jeweils bezogen auf das Gewicht des gesamten Mittels, enthalten.

Als cyclisches, organisches Carbonat der Komponente (b)(i) eignet sich erfindungsgemäß bevorzugt mindestens ein cyclischer Kohlensäureester. Diese cyclischen Ester der Kohlensäure leiten sich vom 1,3-Dioxolan-2-on ab und lassen sich durch folgende Grundstruktur der Formel (II-1) beschreiben: 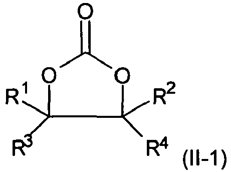 worin die Reste R¹, R², R³ und R⁴ unabhängig voneinander für ein Wasserstoffatom oder organische Reste, insbesondere Alkyl-, Alkenyl- oder Alkylaryl, stehen, die zusätzlich mit weiteren Gruppen, insbesondere Hydroxygruppen, substituiert sein können.

Im Grundkörper, dem 1,3-Dioxolan-2-on, stehen die Reste R¹, R², R³ und R⁴ der Formel (II-1) jeweils für ein Wasserstoffatom. Weiterhin bevorzugt geeignete cyclische Kohlensäureester betreffen Derivate dieses Grundkörpers, wobei mindestens einer der Reste R¹, R², R³ und R⁴ der Formel (II-1) von einem Wasserstoffatom verschieden ist. Hierbei sind der strukturellen Vielfalt keine Grenzen gesetzt, so dass sich mono-, di-, tri- und tetra-substituierte 1,3-Dioxolan-2-one der Formel (II-1) zum Einsatz im Rahmen der vorliegenden Erfindung eignen.

Besonders bevorzugt sind neben dem unsubstituierten 1,3-Dioxolan-2-on insbesondere die in 4-Stellung monosubstituierten Derivate der nachstehenden Formel (II-2) 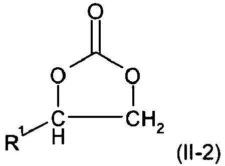 in der R¹ für einen substituierten oder unsubstituierten Alkyl-, Alkenyl- oder Alkylarylrest steht. Bevorzugte Reste R¹ gemäß Formel (II-2) sind Methyl-, Ethyl-, n-Propyl-, iso-Propyl- sowie Hydroxymethyl-, 1-Hydroxyethyl- und 2-Hydroxyethyl-Reste.

Besonders bevorzugte erfindungsgemäße Mittel sind folglich dadurch gekennzeichnet, dass sie als 1,3-Dioxolan-2-on-Derivat mindestens eine Verbindung der obigen Formel (II-2) enthalten, bei der R¹ für einen substituierten oder unsubstituierten Alkyl-, Alkenyl- oder Alkylarylrest steht, wobei in weiter bevorzugten erfindungsgemäßen Mitteln der Rest R¹ in Formel (II-2) ausgewählt ist aus Methyl-, Ethyl-, n-Propyl-, iso-Propyl- sowie Hydroxymethyl-, 1-Hydroxyethyl- und 2-Hydroxyethyl-Resten.

Besonders bevorzugte 1,3-Dioxolan-2-one der Formel (II-1) stammen aus der Gruppe Ethylencarbonat (R¹, R², R³ und R⁴ = H), Propylencarbonat (R¹ = CH₃ sowie R², R³ und R⁴ = H) und Glycerincarbonat (R¹ = CH₂OH sowie R², R³ und R⁴ = H). Ganz besonders bevorzugt eignet sich Propylencarbonat.

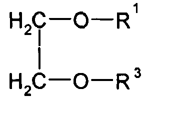

Als Komponente (b)(ii) eignen sich im Rahmen der Erfindung bevorzugt mindestens eine Verbindung der Formel (IV) 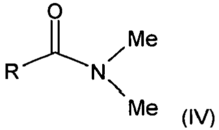 worin
R eine lineare oder verzweigte (C₄ bis C₁₂)-Alkylgruppe bedeutet.
R steht besonders bevorzugt für n-Butyl, sec-Butyl, n-Hexyl, 2-Ethylhexyl, n-Octyl, n-Decyl und n-Dodecyl.

Die Verbindungen der Komponente (b) sind in dem erfindungsgemäßen Mittel bevorzugt in einer Menge von 5 Gew.-% bis 50 Gew.%, insbesondere von 10 Gew.-% bis 30 Gew.%, jeweils bezogen auf das Gewicht des Mittels.

Die Gewichtsverhältnisse der Verbindungen der Formel (I) zu den Verbindungen der Komponente (b) betragen bevorzugt 1 zu 2 bis 1 zu 20, insbesondere 1 zu 3 bis 1 zu 10.

Besonders bevorzugt ist erfindungsgemäß neben der Gegenwart der oben genannten Komponenten (c) und (d) mindestens einer der nachfolgenden Wirkstoffkombinationen in dem erfindungsgemäßen Mittel enthalten:

| | Komponente (a) | Komponente (b) |
|---|---|---|
| 1. | Cystein und/oder ein Salz davon | mindestens eine Verbindung der Formel (II-1) |
| 2. | Cystein und/oder ein Salz davon | mindestens eine Verbindung der Formel (II-2) |
| 3. | Cystein und/oder ein Salz davon | Ethylencarbonat |
| 4. | Cystein und/oder ein Salz davon | Glycerincarbonat |
| 5. | Cystein und/oder ein Salz davon | Propylencarbonat |
| 6. | Cystein und/oder ein Salz davon | mindestens eine Verbindung der Formel (IV) |

Eine weitere Verbesserung der Entfärbekraft stellt sich ein, wenn die erfindungsgemäßen Mittel neben der Wirkstoffkombination zusätzlich Oxalsäure enthalten. Dieser Zusatz erfolgt bevorzugt in einer Menge von 1 Gew.-% bis 5 Gew.-% bezogen auf das Gewicht des Mittels.

Dabei sind folgende Wirkstoffkombinationen in Gegenwart der oben genannten Komponenten (c) und (d) bevorzugt:

| | Komponente (a) | Komponente (b) | |
|---|---|---|---|
| 1. | Cystein und/oder ein Salz davon | mindestens eine Verbindung der Formel (II-1) | Oxalsäure |
| 2. | Cystein und/oder ein Salz davon | mindestens eine Verbindung der Formel (II-2) | Oxalsäure |
| 3. | Cystein und/oder ein Salz davon | Ethylencarbonat | Oxalsäure |
| 4. | Cystein und/oder ein Salz davon | Glycerincarbonat | Oxalsäure |
| 5. | Cystein und/oder ein Salz davon | Propylencarbonat | Oxalsäure |
| 6. | Cystein und/oder ein Salz davon | mindestens eine Verbindung der Formel (IV) | Oxalsäure |

Als Komponente (c) enthält das erfindungsgemäße Mittel zwingend mindestens einen (C₁₀ bis C₃₀)-Fettalkohol, insbesondere mindestens einen (C₁₂ bis C₂₂)-Fettalkohol.

Es ist erfindungsgemäß bevorzugt, dass die Verbindungen der Komponente (c) in einer Menge von 3 bis 10 Gew.-%, besonders bevorzugt von 4 bis 6 Gew.-%, jeweils bezogen auf das Gewicht des erfindungsgemäßen Mittels, in dem erfindungsgemäßen Mittel enthalten sind.

Unter (C₁₀ bis C₃₀)-Fettalkoholen sind primäre aliphatische Alkohole der Formel

R-OH

zu verstehen, in der R für einen aliphatischen, linearen oder verzweigten Kohlenwasserstoffrest mit 10 bis 30 Kohlenstoffatomen (bevorzugt mit 12 bis 18 Kohlenstoffatomen) der gesättigt ist oder bis zu 3 Doppelbindungen enthalten kann, steht.

Typische Beispiele sind Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen.

Bevorzugt sind technische Fettalkoholmischungen mit 12 bis 18 Kohlenstoffatomen wie beispielsweise Kokos-, Palm-, Palmkern- oder Talgfettalkohol, insbesondere Kokos- und/ oder Talgfettalkohol.

Die (C₁₀ bis C₃₀)-Fettalkohole (bevorzugt die (C₁₂ bis C₁₈)-Fettalkohole) sind in dem erfindungsgemäßen Mittel bevorzugt in einer Menge von 3 bis 10 Gew.-%, besonders bevorzugt von 4 bis 6 Gew.-%, jeweils bezogen auf das Gewicht des erfindungsgemäßen Mittels, enthalten. Ferner enthalten die erfindungsgemäßen Mittel als Komponente (d) mindestens einen der oben definierten Emulgatoren. Diese sind bevorzugt in einer Menge von 0,8 bis 7,0 Gew.-%, besonders bevorzugt von 1,0 bis 2,5 Gew.-%, jeweils bezogen auf das Gewicht des Mittels, enthalten.

Die Verbindungen der Komponente (c) und die Verbindungen der Komponente (d) sind erfindungsgemäß bevorzugt in einem Gewichtsverhältnis von 5 zu 1 bis 20 zu 1, besonders bevorzugt von 8 zu 1 bis 12 zu 1, in dem erfindungsgemäßen Mittel enthalten.

Darüberhinaus enthalten die erfindungsgemäßen Mittel als Komponente (d) mindestens einen Emulgator ausgewählt aus mit mindestens einem (C₁₀ bis C₃₀)- Kohlenwasserstoffrest substituierten amphoteren Emulgator. Unter einem (C₁₀ bis C₃₀)-Kohlenwasserstoffrest werden lineare oder verzweigte, gesättigte oder ungesättigte Kohlenwasserstoffketten mit (C₁₀ bis C₃₀)-Kohlenstoffatomen verstanden.

Die amphoteren Emulgatoren der Komponente (d) sind in dem erfindungsgemäßen Mittel bevorzugt in einer Menge von 0,5 bis 5,0 Gew.%, besonders bevorzugt von 1,0 bis 2,0 Gew.-%, jeweils bezogen auf das Gewicht des erfindungsgemäßen Mittels, enthalten.

Amphotere Emulgatoren unterteilen sich in die Gruppen der zwitterionischen Emulgatoren und der ampholytischen Emulgatoren, wobei sich erfindungsgemäß die zwitterionischen Emulgatoren bevorzugt eignen.

Als zwitterionische Emulgatoren werden solche oberflächenaktiven Verbindungen bezeichnet, die im Molekül mindestens eine quartäre Ammoniumgruppe und mindestens eine -COO⁽⁻⁾ - oder -SO₃⁽⁻⁾ -Gruppe tragen.

Darunter eignen sich erfindungsgemäß beispielsweise die sogenannten Sulfobetaine, wie beispielsweise N-(C₁₀ bis C₃₀)-Acylaminoethyl-N,N-dimethyltaurin, N-(C₁₀ bis C₃₀)-Acylaminopropyl-N,N-dimethyltaurin, N-(C₁₀ bis C₃₀)-Acylaminoethyl-N,N-dimethylsarcosin, N-(C₁₀ bis C₃₀)-Acylaminopropyl-N,N-dimethylsarcosin.

Besonders geeignete zwitterionische Emulgatoren sind die sogenannten Betaine wie die N-Alkyl-N,N-dimethylammonium-glycinate, beispielsweise das Kokosalkyl-dimethylammoniumglycinat, N-(C₁₀ bis C₃₀)-Acylaminoethyl-N,N-dimethylammoniumglycinate (beispielsweise das Kokosacylaminoethyl-dimethylammoniumglycinat), N-(C₁₀ bis C₃₀)-Acylaminopropyl-N,N-dimethylammoniumglycinate (beispielsweise das Kokosacylaminopropyldimethylammoniumglycinat) und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat.

Bevorzugt geeignete zwitterionische Emulgatoren werden ausgewählt aus mindestens einer Betainverbindung aus N-(N'-(C₁₀ bis C₃₀)-Acylamino-(C₂ oder C₃)-alkyl)-N,N-dialkylglycin der Formel (D-1), 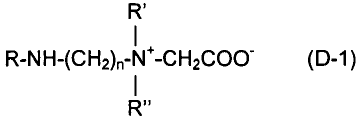 worin
- R: für eine (C₁₀ bis C₃₀)-Acylgruppe steht,
- R' und R": unabhängig voneinander für eine Methyl- oder eine Ethylgruppe steht (insbesondere stehen R' und R" für eine Methylgruppe),
- n: steht für die Zahl 2 oder 3 (bevorzugt für 3).

Ebenso eignen sich die mit den Verbindungen der Formel (D-1) verwandten Sulfobetaine der Formel (D1-a) zur erfindungsgemäßen Verwendung als Komponente (d), führen allerdings nicht in dem gesteigerten Maße zu den erfindungsgemäßen Effekten, wie sie unter Einsatz der Betaine der Formel (D-1) erhalten werden 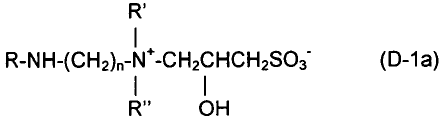 worin
- R: für eine (C₁₀ bis C₃₀)-Acylgruppe steht,
- R' und R": unabhängig voneinander für eine Methyl- oder eine Ethylgruppe steht (insbesondere stehen R' und R" für eine Methylgruppe),
- n: steht für die Zahl 2 oder 3 (bevorzugt für 3).

Ein besonders bevorzugtes zwitterionisches Tensid ist das unter der INCI-Bezeichnung Cocamidopropyl Betaine bekannte Fettsäureamid-Derivat, welches beispielsweise unter dem Handelsnamen Dehyton K bzw. Dehyton PK 45 der Fa. Cognis gehandelt wird.

Weiterhin als amphotere Emulgatoren (d) geeignet sind ampholytische Emulgatoren - diese sind jedoch nicht bevorzugte amphotere Emulgatoren der erfindungsgemäßen Komponente (d). Unter ampholytischen Emulgatoren werden solche oberflächenaktiven Verbindungen verstanden, die außer mindestens einer C₁₀-C₃₀-Alkyl- oder Acylgruppe im Molekül mindestens eine freie Aminogruppe und mindestens eine -CO-H- oder -SO_H-Gruppe enthalten und zur Ausbildung innerer Salze befähigt sind.

Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine (insbesondere diejenigen der nachfolgenden Formel (D-2)), N-Alkylsarcosine (insbesondere diejenigen der nachfolgenden Formel (D-3)), 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat, N-C₁₂₋₁₈-Acyltaurine und C₁₂₋₁₈-Acylsarcosine.

Bevorzugte ampholytische Tenside werden ausgewählt aus mindestens einem nachstehenden N-Acylsarcosin-Derivat der Formel (D-2) und/oder mindestens einem N-Acyltaurin-Derivat der Formel (D-3) 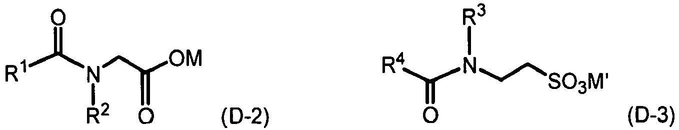 worin
R¹ eine (C₇ bis C₃₀)-Alkylgruppe, (C₇ bis C₃₀)-Alkenylgruppe oder eine
(C₇ bis C₃₀)-Hydroxyalkylgruppe bedeutet,
R² eine (C₁ bis C₄)-Alkylgruppe oder eine (C₁ bis C₄)-Hydroxyalkylgruppe bedeutet,
R³ für ein Wasserstoffatom, eine (C₁ bis C₄)-Alkylgruppe oder eine (C₁ bis C₄)-Hydroxyalkylgruppe steht,
R⁴ für eine (C₇ bis C₃₀)-Alkylgruppe, (C₇ bis C₃₀)-Alkenylgruppe oder eine
(C₇ bis C₃₀)-Hydroxyalkylgruppe steht und
M und M' unabhängig voneinander für ein Wasserstoffatom oder ein Äquivalent eines ein- oder mehrwertigen Kations stehen.

Die Reste R¹ der Formel (D-2) und R⁴ der Formel (D-3) stehen bevorzugt unabhängig voneinander für eine (C₉ bis C₂₃)-Alkylgruppe, (C₉ bis C₂₃)-Alkenylgruppe oder eine (C₉ bis C₂₃)-Hydroxyalkylgruppe, besonders bevorzugt unabhängig voneinander für einen Rest, ausgewählt aus der Liste, die gebildet wird, aus Nonyl, Undecyl, Tridecyl, Pentadecyl, Heptadecyl, Nonadecyl, Henicosanyl, 15-Methylhexadecyl, Heptadec-8-enyl, Heptadeca-8,11-dienyl, Nonadeca-4,7,10,13-tetraenyl, Heptadeca-8,11,14-trienyl und 11-Hydroxyheptadecyl.

Der Rest R² gemäß Formel (D-2) steht bevorzugt für einen Rest, ausgewählt aus der Gruppe, die gebildet wird aus Methyl, Ethyl, Isopropyl, n-Propyl und 2-Hydroxyethyl. Besonders bevorzugt steht R² der Formel (D-2) für ein Wasserstoffatom oder eine Methylgruppe.

Der Rest R³ gemäß Formel (D-3) steht bevorzugt für einen Rest, ausgewählt aus der Gruppe, die gebildet wird aus Wasserstoffatom, Methyl, Ethyl, Isopropyl, n-Propyl und 2-Hydroxyethyl.

Wenn die Verbindungen der Formeln (D-2) bzw. (D-3) als Säure vorliegen, bedeutet der Rest M bzw. der Rest M' ein Wasserstoffatom. Wenn die Verbindungen der Formeln (D-2) bzw. (D-3) als Salz vorliegen, stehen M bzw. M' für ein Äquivalent eines ein- oder mehrwertigen Kations. Das ein oder mehrwertige Kation M^{z+} bzw. M'^{y+} mit jeweils einer Ladungszahl z bzw. y von eins oder höher dient lediglich aus Gründen der Elektroneutralität zur Kompensation der einfach negativen Ladung des bei Salzbildung vorliegenden Carboxylatfragments -CO-⁽⁻⁾ der Formel (D-2) bzw. des Sulfonatfragments -SO_⁽⁻⁾ der Formel (D-3). Das dafür zu verwendende Äquivalent des entsprechenden Kations beträgt 1/z bzw. 1/y. Das Fragment -CO-M der Formel (D-2) bzw. das Fragment -SO_M der Formel (D-3) steht im Fall der Salzbildung für die Gruppe:

-COO⁽⁻⁾ 1/z (M^{z+}) bzw. -SO₃⁽⁻⁾ 1/y (M'^{y+})

Als ein- oder mehrwertige Kationen M^{z+} bzw. M'^{y+} kommen prinzipiell alle physiologisch verträglichen Kationen in Frage. Insbesondere sind dies Metallkationen der physiologisch verträglichen Metalle aus den Gruppen la, Ib, IIa, IIb, IIIb, VIa oder VIII des Periodensystems der Elemente, Ammoniumionen, sowie kationische organische Verbindungen mit quaterniertem Stickstoffatom. Letztere werden beispielsweise durch Protonierung primärer, sekundärer oder tertiärer organischer Amine mit einer Säure, wie z.B. mit Verbindungen der Formel (D-2) oder Formel (D-3) in ihrer sauren Form, oder durch permanente Quaternisierung besagter organischer Amine gebildet. Beispiele dieser kationischen organischen Ammoniumverbindungen sind 2-Ammonioethanol und 2-Trimethylammonioethanol. M bzw. M' steht in den Formeln (D-2) bzw. (D-3) bevorzugt für ein Wasserstoffatom, ein Ammoniumion, ein Alkalimetallion, für ein halbes Äquivalent eines Erdalkalimetallions oder ein halbes Äquivalent eines Zinkions, besonders bevorzugt für ein Wasserstoffatom, ein Ammoniumion, ein Natriumion, ein Kaliumion, ½ Kalziumion, ½ Magnesiumion oder ½ Zinkion.

Die Verbindungen der Formel (D-2) werden bevorzugt ausgewählt aus mindestens einem Vertreter aus der Gruppe, die gebildet wird, aus N-Lauroylsarcosin, N-Myristoylsarcosin, N-Palmitoylsarcosin, N-Oleylsarcosin, N-Cocoylsarcosin (wobei hier eine Gruppe von Verbindungen vorliegt und Cocoyl der Zusammensetzung des Fettsäureschnitt des Kokosnussöls entspricht) und N-Palmkernsarcosin (wobei hier eine Gruppe von Verbindungen vorliegt und Palmkern der Zusammensetzung des Fettsäureschnitt des Palmkernöls entspricht), sowie aus den Salzen der vorgenannten N-Acylsarcosinderivate.

Die Verbindungen der Formel (D-3) werden bevorzugt ausgewählt aus mindestens einem Vertreter aus der Gruppe, die gebildet wird, aus N-Dodecanoyl-N-methyltaurin, N-Octadecanoyl-N-methyltaurin, N-Hexadecanoyl-N-methyltaurin, N-Tetradecanoyl-N-methyltaurin, N-Oleyl-N-methyltaurin, N-Cocoyl-N-methyltaurin (wobei hier eine Gruppe von Verbindungen vorliegt und Cocoyl der Zusammensetzung des Fettsäureschnitt des Kokosnussöls entspricht), N-Palmkern-N-methyltaurin (wobei hier eine Gruppe von Verbindungen vorliegt und Palmkern der Zusammensetzung des Fettsäureschnitt des Palmkernöls entspricht), sowie aus den Salzen der vorgenannten N-Acyltaurinderivate.

Besonders bevorzugt ist erfindungsgemäß mindestens einer der nachfolgenden Wirkstoffkombinationen in dem erfindungsgemäßen Mittel enthalten:

| | Komponente (a) | Komponente (b) | Komponente (c) | Komponente (d) |
|---|---|---|---|---|
| 1. | Cystein und/oder ein Salz davon | mindestens eine Verbindung der Formel (II-1) | mindestens einen (C₁₂ bis C₁₈)-Fettalkohol | mindestens einer Betainverbindung der Formel (D-1) |
| 2. | Cystein und/oder ein Salz davon | mindestens eine Verbindung der Formel (II-2) | mindestens einen (C₁₂ bis C₁₈)-Fettalkohol | mindestens einer Betainverbindung der Formel (D-1) |
| 3. | Cystein und/oder ein Salz davon | Ethylencarbonat | mindestens einen (C₁₂ bis C₁₈)-Fettalkohol | mindestens einer Betainverbindung der Formel (D-1) |
| 4. | Cystein und/oder ein Salz davon | Glycerincarbonat | mindestens einen (C₁₂ bis C₁₈)-Fettalkohol | mindestens einer Betainverbindung der Formel (D-1) |
| 5. | Cystein und/oder ein Salz davon | Propylencarbonat | mindestens einen (C₁₂ bis C₁₈)-Fettalkohol | mindestens einer Betainverbindung der Formel (D-1) |
| 6. | Cystein und/oder ein Salz davon | mindestens eine Verbindung der Formel (IV) | mindestens einen (C₁₂ bis C₁₈)-Fettalkohol | mindestens einer Betainverbindung der Formel (D-1) |

Insbesondere die erfindungsgemäß bevorzugten Parameter der Viskosität und des pH-Wertes, sowie diejenigen der Verbindungen der Formeln (II-1), (II-2), (IV) und (D-1) gelten auch für diese Ausführungsformen der Erfindung.

Als Träger für ein anwendungsbereites Mittel eignen sich bevorzugt flüssige Medien, wie beispielsweise Wasser oder organische Lösemittel, die verschieden von den Komponenten des erfindungsgemäßen Wirkstoffkomplexes sind. Es ist erfindungsgemäß bevorzugt, wenn der Träger ein kosmetischer Träger ist.

Als kosmetische Träger eignen sich besonders Cremes, Emulsionen, Gele oder auch tensidhaltige schäumende Lösungen, wie beispielsweise Shampoos, Schaumaerosole oder andere Zubereitungen, die insbesondere für die Anwendung auf dem Haar geeignet sind. Es ist aber auch denkbar, die Inhaltsstoffe in eine pulverförmige oder auch tablettenförmige Formulierung zu integrieren, welche vor der Anwendung in Wasser gelöst wird. Die kosmetischen Träger können insbesondere wässrig oder wässrig-alkoholisch sein.

Ein wässriger kosmetischer Träger enthält mindestens 50 Gew.-% Wasser.

Unter wässrig-alkoholischen kosmetischen Trägern sind im Sinne der vorliegenden Erfindung wässrige Lösungen enthaltend 3 bis 70 Gew.-% eines von den Verbindungen der Komponente (b) verschiedenen C₁-C₈-Alkohols, insbesondere Ethanol bzw. Isopropanol, zu verstehen. Weitere alkoholische Lösemittel, sind beispielsweise Methoxybutanol, Benzylalkohol, 2-Phenoxyethanol, Ethyldiglykol oder 1,2-Propylenglykol. In einer weiteren Ausführungsform enthält das erfindungsgemäße Mittel zusätzlich als Lösemittel mindestens einen (C₂ bis C₆)-Alkylmonoalkohol und/oder ein (C₂ bis C₆)-Alkandiol, insbesondere Ethanol, Isopropanol und/oder 1,2-Propylenglykol.

Es ist ebenso erfindungsgemäß bevorzugt, wenn das erfindungsgemäße Mittel zusätzlich zur Effektsteigerung mindestens ein Redukton enthält. Unter einem Redukton versteht der Fachmann reduktiv wirkende Endiol-Verbindungen, die durch Substitution in α-Stellung stabilisiert sind und die der Tautomerie unterliegen. Die wichtigsten erfindungsgemäß einsetzbaren Reduktone sind Ascorbinsäure, Isoascorbinsäure, 2,3-Dihydroxy-2-propendial und 2,3-Dihydroxy-2-cyclopentenon.

Die Reduktone sind bevorzugt in einer Menge von 1,0 bis 5,0 Gew.-% bezogen auf das Gewicht des Mittels, in dem erfindungsgemäßen Mittel enthalten. Dabei sind folgende Wirkstoffkombinationen neben der Gegenwart der Komponenten (c) und (d) bevorzugt:

| | Komponente (a) | Komponente (b) | Redukton |
|---|---|---|---|
| 1. | Cystein und/oder ein Salz davon | mindestens eine Verbindung der Formel (II-1) | Ascorbinsäure |
| 2. | Cystein und/oder ein Salz davon | mindestens eine Verbindung der Formel (II-2) | Ascorbinsäure |
| 3. | Cystein und/oder ein Salz davon | Ethylencarbonat | Ascorbinsäure |
| 4. | Cystein und/oder ein Salz davon | Glycerincarbonat | Ascorbinsäure |
| 5. | Cystein und/oder ein Salz davon | Propylencarbonat | Ascorbinsäure |
| 6. | Cystein und/oder ein Salz davon | mindestens eine Verbindung der Formel (IV) | Ascorbinsäure |
| 7. | Cystein und/oder ein Salz davon | mindestens eine Verbindung der Formel (II-1) | Isoascorbinsäure |
| 8. | Cystein und/oder ein Salz davon | mindestens eine Verbindung der Formel (II-2) | Isoascorbinsäure |
| 9. | Cystein und/oder ein Salz davon | Ethylencarbonat | Isoascorbinsäure |
| 10. | Cystein und/oder ein Salz davon | Glycerincarbonat | Isoascorbinsäure |
| 11. | Cystein und/oder ein Salz davon | Propylencarbonat | Isoascorbinsäure |
| 12. | Cystein und/oder ein Salz davon | mindestens eine Verbindung der Formel (IV) | Isoascorbinsäure |

Ferner steigert sich die Leistung der erfindungsgemäßen Mittel, wenn sie zusätzlich mindestens eine Oxocarbonsäure enthalten. Oxocarbonsäuren sind organische Verbindungen, die neben mindestens einer Carboxylgruppe eine Carbonylgruppe tragen und sind somit Aldehyd- bzw. Ketocarbonsäuren. Bevorzugte Oxocarbonsäuren sind α-Oxocarbonsäuren, β-Oxocarbonsäuren, γ-Oxocarbonsäuren sowie ω-Oxocarbonsäuren. Darunter sind wiederum Verbindungen der Formel (V) bzw. deren Salze bevorzugt, 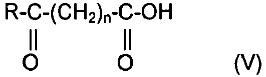 worin bedeuten
- R: ein Wasserstoffatom, eine (C₁ bis C₆)-Alkylgruppe, eine (C₁ bis C₆)-Hydroxyalkylgruppe, eine gegebenenfalls substituierte Arylgruppe, eine gegebenenfalls substituierte Heteroarylgruppe, eine (C₂ bis C₆)-Alkenylgruppe oder eine Carboxy-(C₁ bis C₆)-alkylgruppe,
- n: eine Zahl 0, 1, 2 oder 3.

Besonders bevorzugt werden die Oxocarbonsäuren ausgewählt aus mindestens einem Vertreter aus der Gruppe Glyoxalsäure, Acetessigsäure, 3-Oxoglutarsäure, 4-Oxovaleriansäure und Brenztraubensäure bzw. den Salzen der vorgenannten Säuren.

Dabei sind folgende Wirkstoffkombinationen neben der Gegenwart der Komponenten (c) und (d) bevorzugt:

| | Komponente (a) | Komponente (b) | Oxocarbonsäure |
|---|---|---|---|
| 1. | Cystein und/oder ein Salz davon | mindestens eine Verbindung der Formel (II-1) | Glyoxalsäure |
| 2. | Cystein und/oder ein Salz davon | mindestens eine Verbindung der Formel (II-2) | Glyoxalsäure |
| 3. | Cystein und/oder ein Salz davon | Ethylencarbonat | Glyoxalsäure |
| 4. | Cystein und/oder ein Salz davon | Glycerincarbonat | Glyoxalsäure |
| 5. | Cystein und/oder ein Salz davon | Propylencarbonat | Glyoxalsäure |
| 6. | Cystein und/oder ein Salz davon | mindestens eine Verbindung der Formel (IV) | Glyoxalsäure |
| 7. | Cystein und/oder ein Salz davon | mindestens eine Verbindung der Formel (II-1) | 3-Oxoglutarsäure |
| 8. | Cystein und/oder ein Salz davon | mindestens eine Verbindung der Formel (II-2) | 3-Oxoglutarsäure |
| 9. | Cystein und/oder ein Salz davon | Ethylencarbonat | 3-Oxoglutarsäure |
| 10. | Cystein und/oder ein Salz davon | Glycerincarbonat | 3-Oxoglutarsäure |
| 11. | Cystein und/oder ein Salz davon | Propylencarbonat | 3-Oxoglutarsäure |
| 12. | Cystein und/oder ein Salz davon | mindestens eine Verbindung der Formel (IV) | 3-Oxoglutarsäure |

Die Oxocarbonsäuren sind bevorzugt in einer Menge von 1,0 bis 5,0 Gew.-% bezogen auf das Gewicht des Mittels, in dem erfindungsgemäßen Mittel enthalten.

Die in dem erfindungsgemäßen Verfahren eingesetzten kosmetischen Mittel können weiterhin alle für solche Zubereitungen bekannten Wirk-, Zusatz- und Hilfsstoffe enthalten:
Das Nachwaschen mit einem Shampoo entfällt, wenn ein stark tensidhaltiger Träger verwendet wurde.

In vielen Fällen enthalten die Mittel zusätzlich mindestens ein Tensid, ausgewählt aus einem Vertreter aus der Gruppe, die gebildet wird aus anionischen, nichtionischen und kationischen Tenside.

Als anionische Tenside eignen sich in den kosmetischen Mitteln alle für die Verwendung am menschlichen Körper geeigneten anionischen oberflächenaktiven Stoffe. Diese sind gekennzeichnet durch eine wasserlöslichmachende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-CO-H, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialky-ester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- anionische Alkyloligoglykoside bzw. anionische Alkenyloligoglykosid-Derivate, ausgewählt aus Alkyl- und/oder Alkenyl-Oligoglykosidcarboxylaten, -sulfaten, - phosphaten und/oder -isethi-naten, die sich von Alkyl- und/oder Alkenyloligoglykosiden der allgemeinen Formel (VI) ableiten,

   R-O-(G)ₚ (VI)

   mit der Bedeutung
   - R: C₆₋₂₂-Alkyl oder C₆₋₂₂-Alkenyl,
   - G: Glykosideinheit, die sich von einem Zucker mit 5 oder 6 Kohlenstoffatomen ableitet,
   - p: Zahl von 1 bis 10,
   insbesondere das Laurylglucosidcarboxylat, wie es als Plantapon^{®} LGC von Cognis Deutschland erhältlich ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Erfindungsgemäß werden als kationische Tenside insbesondere solche vom Typ der quartären Ammoniumverbindungen, der Esterquats und der Amidoamine eingesetzt.

Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalze von Fettsäuren mit 1,2-Dihydroxypropyldialkylaminen. Solche Produkte werden beispielsweise unter den Warenzeichen Stepantex^{®}, Dehyquart^{®} und Armocare^{®} vertrieben. Die Produkte Armocare^{®} VGH-70, ein N,N-Bis(2-Palmitoyloxyethyl)dimethylammoniumchlorid, sowie Dehyquart^{®} F-75 und Dehyquart^{®} AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid^{®} S 18 im Handel erhältliche Stearamidopropyl-dimethylamin dar.

Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quaternium-80).

Ein Beispiel für ein als kationisches Tensid einsetzbares quaternäres Zuckerderivat stellt das Handelsprodukt Glucquat^{®}100 dar, gemäß INCI-Nomenklatur ein "Lauryl"Methyl Gluceth-10 Hydroxypropyl Dimonium Chloride".

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, - hydroxide oder -alkoholate als Katalysatoren verwendet werden. Die Verwendung von Produkten mit eingeengter Homologenverteilung kann bevorzugt sein.

Die erfindungsgemäßen Mittel können zur Verstärkung der Faserpflege ohne Reduzierung der Entfärbekraft zusätzlich mindestens ein Silikon enthalten. Die Silikone, wenn sie in den erfindungsgemäßen Mitteln zugegen sind, vorzugsweise in Mengen von 0,05 bis 5 Gew.-%, vorzugsweise von 0,2 bis 5 Gew.-%, jeweils bezogen auf das anwendungsbereite Mittel, enthalten.

Insbesondere bevorzugt werden die Silikone ausgewählt, aus mindestens einem Vertreter aus der Liste, die gebildet wird aus:
(i) Polyalkylsiloxanen, Polyarylsiloxanen, Polyalkylarylsiloxanen, die flüchtig oder nicht flüchtig, geradkettig, verzweigt oder cyclisch, vernetzt oder nicht vernetzt sind;
(ii) Polysiloxanen, die in ihrer allgemeinen Struktur eine oder mehrere organofunktionelle Gruppen enthalten, die ausgewählt sind unter:
   a) substituierten oder unsubstituierten aminierten Gruppen;
   b) (per)fluorierten Gruppen;
   c) Thiolgruppen;
   d) Carboxylatgruppen;
   e) hydroxylierten Gruppen;
   f) alkoxylierten Gruppen;
   g) Acyloxyalkylgruppen;
   h) amphoteren Gruppen;
   i) Bisulfitgruppen;
   j) Hydroxyacylaminogruppen;
   k) Carboxygruppen;
   l) Sulfonsäuregruppen; und
   m) Sulfat- oder Thiosulfatgruppen;
(iii) linearen Polysiloxan(A)- Polyoxyalkylen(B)- Blockcopoylmeren vom Typ (A-B)ₙ mit n > 3;
(iv) gepfropften Silikonpolymeren mit nicht silikonhaltigem, organischen Grundgerüst, die aus einer organischen Hauptkette bestehen, welche aus organischen Monomeren gebildet wird, die kein Silikon enthalten, auf die in der Kette sowie gegebenenfalls an mindestens einem Kettenende mindestens ein Polysiloxanmakromer gepfropft wurde;
(v) gepfropften Silikonpolymeren mit Polysiloxan-Grundgerüst, auf das nicht silikonhaltige, organische Monomere gepfropft wurden, die eine Polysiloxan-Hauptkette aufweisen, auf die in der Kette sowie gegebenenfalls an mindestens einem ihrer Enden mindestens ein organisches Makromer gepfropft wurde, das kein Silikon enthält, wie beispielsweise das unter der INCI-Bezeichnung Bis-PEG/PPG-20/20 Dimethicone vertriebene Handelsprodukt Abil B 8832 der Firma Degussa;
(vi) oder deren Gemischen.

Erfindungsgemäß geeignet sind kationische Silikonöle wie beispielsweise die im Handel erhältliche Dow Corning 929 Emulsion (enthaltend ein hydroxylamino-modifiziertes Silikon, das als Amodimethicone bezeichnet wird), DC 2-2078 (Hersteller Dow Corning, INCI-Bezeichnung: Aminopropyl Phenyl Trimethicone), DC 5-7113 (Hersteller Dow Corning, INCI-Bezeichnung: Silicone Quaternium 16), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quaternium-80). Geeignet sind auch Silikone, die nach der INCI-Deklaration als Trimethylsilylamodimethicone bezeichnet werden. Diese sind beispielsweise unter der Bezeichnung Q2-7224 (Hersteller: Dow Corning; ein stabilisiertes Trimethylsilylamodimethicon) erhältlich. Auch Silikone, die nach der INCI-Deklaration als Amodimethicone, bzw. als funktionalisierte Amodimethicone, wie beispielsweise Bis(C13-15 Alkoxy) PG Amodimethicone (beispielsweise als Handelsprodukt: DC 8500 der Firma Dow Corning erhältlich), Trideceth-9 PG-Amodimethicone (beispielsweise als Handelsprodukt Silcare Silicone SEA der Firma Clariant erhältlich) bezeichnet werden, eignen sich. Unabhängig davon, welche aminofunktionellen Silikone eingesetzt werden, sind erfindungsgemäße kosmetische oder dermatologische Zubereitungen bevorzugt, die ein aminofunktionelles Silikon enthalten dessen Aminzahl oberhalb von 0,25 meq/g, vorzugsweise oberhalb von 0,3 meq/g und insbesondere oberhalb von 0,4 meq/g liegt. Die Aminzahl steht dabei für die Milli-Äquivalente Amin pro Gramm des aminofunktionellen Silikons. Sie kann durch Titration ermittelt und auch in der Einheit mg KOH/g angegeben werden.

Erfindungsgemäß bevorzugte kosmetische oder dermatologische Zubereitungen sind dadurch gekennzeichnet, dass sie, bezogen auf ihr Gewicht, 0,01 bis 10 Gew.%, vorzugsweise 0,1 bis 8 Gew.%, besonders bevorzugt 0,25 bis 7,5 Gew.% und insbesondere 0,5 bis 5 Gew.% aminofunktionelle(s) Silikon(e) enthalten.

Auch die nach INCI als Cyclomethicone bezeichneten cyclischen Dimethicone sind erfindungsgemäß mit Vorzug einsetzbar. Hier sind erfindungsgemäße kosmetische oder dermatologische Zubereitungen bevorzugt, die mindestens ein Silikon der Formel (Si-4) 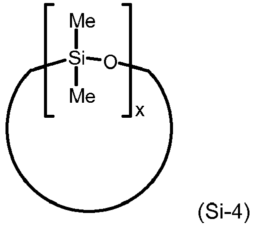 enthalten, in der x für eine Zahl von 0 bis 200, vorzugsweise von 0 bis 10, weiter bevorzugt von 0 bis 7 und insbesondere 0, 1, 2, 3, 4, 5 oder 6, steht.

Die vorstehend beschriebenen Silikone weisen ein Rückgrat auf, welches aus -Si-O-S--Einheiten aufgebaut ist. Selbstverständlich können diese Si-O-Si-Einheiten auch durch Kohlenstoffketten unterbrochen sein. Entsprechende Moleküle sind durch Kettenverlängerungsreaktionen zugänglich und kommen vorzugsweise in Form von Silikon-in-Wasser-Emulsionen zum Einsatz.

Desweiteren enthält das erfindungsgemäße Mittel bevorzugt zur Haarkonditionierung mindestens ein kationisches Polymer. Erfindungsgemäße Mittel, die ein solches Polymer enthalten, erleiden keinen Leistungsabfall der Entfärbekraft, sondern erfahren sogar eine leichte Wirkungssteigerung.

Unter kationischen Polymeren sind erfindungsgemäß Polymere zu verstehen, welche in der Haupt- und/oder Seitenkette eine Gruppe aufweisen, welche "temporär" oder "permanent" kationisch sein kann. Als "permanent kationisch" werden erfindungsgemäß solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine C₁-₄-Kohlenwasserstoffgruppe an eine aus Acrylsäure, Methacrylsäure oder deren Derivaten aufgebaute Polymerhauptkette gebunden sind, haben sich als besonders geeignet erwiesen. Homopolymere der allgemeinen Formel (G1-I),

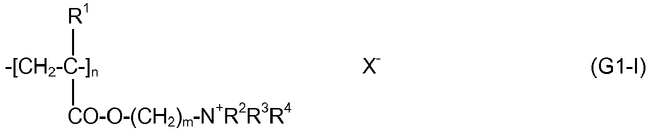

in der R¹= -H oder -CH₃ ist, R², R³ und R⁴ unabhängig voneinander ausgewählt sind aus C₁₋₄-Alkyl-, -Alkenyl- oder -Hydroxyalkylgruppen, m = 1, 2, 3 oder 4, n eine natürliche Zahl und X⁻ ein physiologisch verträgliches organisches oder anorganisches Anion ist, sowie Copolymere, bestehend im wesentlichen aus den in Formel (G1-I) aufgeführten Monomereinheiten sowie nichtionogenen Monomereinheiten, sind besonders bevorzugte kationische Polymere. Im Rahmen dieser Polymere sind diejenigen erfindungsgemäß bevorzugt, für die mindestens eine der folgenden Bedingungen gilt:
R¹ steht für eine Methylgruppe
R², R³ und R⁴ stehen für Methylgruppen
m hat den Wert 2.

Als physiologisch verträgliches Gegenionen X⁻ kommen beispielsweise Halogenidionen, Sulfationen, Phosphationen, Methosulfationen sowie organische Ionen wie Lactat-, Citrat-, Tartrat- und Acetationen in Betracht. Bevorzugt sind Halogenidionen, insbesondere Chlorid.

Ein besonders geeignetes Homopolymer ist das, gewünschtenfalls vernetzte, Poly(methacryloyloxyethyltrimethylammoniumchlorid) mit der INCI-Bezeichnung Polyquaternium-37. Die Vernetzung kann gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen. Methylenbisacrylamid ist ein bevorzugtes Vernetzungsagens.

Das Homopolymer wird bevorzugt in Form einer nichtwäßrigen Polymerdispersion, die einen Polymeranteil nicht unter 30 Gew.-% aufweisen sollte, eingesetzt. Solche Polymerdispersionen sind unter den Bezeichnungen Salcare^{®} SC 95 (ca. 50 % Polymeranteil, weitere Komponenten: Mineralöl (INCI-Bezeichnung: Mineral Oil) und Tridecyl-polyoxypropylen-polyoxyethylen-ether (INCI-Bezeichnung: PPG-1-Trideceth-6)) und Salcare^{®} SC 96 (ca. 50 % Polymeranteil, weitere Komponenten: Mischung von Diestern des Propylenglykols mit einer Mischung aus Capryl- und Caprinsäure (INCI-Bezeichnung: Propylene Glycol Dicaprylate/Dicaprate) und Tridecyl-polyoxypropylen-polyoxyethylen-ether (INCI-Bezeichnung: PPG-1-Trideceth-6)) im Handel erhältlich.

Copolymere mit Monomereinheiten gemäß Formel (G1-I) enthalten als nichtionogene Monomereinheiten bevorzugt Acrylamid, Methacrylamid, Acrylsäure-C₁₋₄-alkylester und Methacrylsäure-C₁₋₄-alkylester. Unter diesen nichtionogenen Monomeren ist das Acrylamid besonders bevorzugt. Auch diese Copolymere können, wie im Falle der Homopolymere oben beschrieben, vernetzt sein. Ein erfindungsgemäß bevorzugtes Copolymer ist das vernetzte Acrylamid-Methacryloyloxyethyltrimethylammoniumchlorid-Copolymer. Solche Copolymere, bei denen die Monomere in einem Gewichtsverhältnis von etwa 20:80 vorliegen, sind im Handel als ca. 50 %ige nichtwäßrige Polymerdispersion unter der Bezeichnung Salcare^{®} SC 92 erhältlich.

Weitere bevorzugte kationische Polymere sind beispielsweise
- quaternisierte Cellulose-Derivate, wie sie unter den Bezeichnungen Celquat^{®} und Polymer JR^{®} im Handel erhältlich sind. Die Verbindungen Celquat^{®} H 100, Celquat^{®} L 200 und Polymer JR^{®}400 sind bevorzugte quaternierte Cellulose-Derivate,
- kationische Alkylpolyglycoside gemäß der DE-PS 44 13 686,
- kationisierter Honig, beispielsweise das Handelsprodukt Honeyquat^{®} 50,
- kationische Guar-Derivate, wie insbesondere die unter den Handelsnamen Cosmedia^{®}Guar und Jaguar^{®} vertriebenen Produkte,
- Polysiloxane mit quaternären Gruppen, wie beispielsweise die im Handel erhältlichen Produkte Q2-7224 (Hersteller: Dow Corning; ein stabilisiertes Trimethylsilylamodimethicon), Dow Corning^{®} 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt), diquaternäre Polydimethylsiloxane, Quaternium-80),
- polymere Dimethyldiallylammoniumsalze und deren Copolymere mit Estern und Amiden von Acrylsäure und Methacrylsäure. Die unter den Bezeichnungen Merquat^{®}100 (Poly(dimethyldiallylammoniumchlorid)) und Merquat^{®}550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und -methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat^{®}734 und Gafquat^{®}755 im Handel erhältlich,
- Vinylpyrrolidon-Vinylimidazoliummethochlorid-Copolymere, wie sie unter den Bezeichnungen Luviquat^{®} FC 370, FC 550, FC 905 und HM 552 angeboten werden,
- quaternierter Polyvinylalkohol,
- sowie die unter den Bezeichnungen Polyquaternium 2, Polyquaternium 17, Polyquaternium 18 und Polyquaternium 27 bekannten Polymeren mit quartären Stickstoffatomen in der Polymerhauptkette.

Gleichfalls als kationische Polymere eingesetzt werden können die unter den Bezeichnungen Polyquaternium-24 (Handelsprodukt z. B. Quatrisoft^{®} LM 200), bekannten Polymere. Ebenfalls erfindungsgemäß verwendbar sind die Copolymere des Vinylpyrrolidons, wie sie als Handelsprodukte Copolymer 845 (Hersteller: ISP), Gaffix^{®} VC 713 (Hersteller: ISP), Gafquat^{®}ASCP 1011, Gafquat^{®}HS 110, Luviquat^{®}8155 und Luviquat^{®} MS 370 erhältlich sind. Weitere erfindungsgemäße kationische Polymere sind die sogenannten "temporär kationischen" Polymere. Diese Polymere enthalten üblicherweise eine Aminogruppe, die bei bestimmten pH-Werten als quartäre Ammoniumgruppe und somit kationisch vorliegt. Bevorzugt sind beispielsweise Chitosan und dessen Derivate, wie sie beispielsweise unter den Handelsbezeichnungen Hydagen^{®} CMF, Hydagen^{®} HCMF, Kytamer^{®} PC und Chitolam^{®} NB/101 im Handel frei verfügbar sind.

Erfindungsgemäß bevorzugte kationische Polymere sind kationische Cellulose-Derivate und Chitosan und dessen Derivate, insbesondere die Handelsprodukte Polymer^{®}JR 400, Hydagen^{®} HCMF und Kytamer^{®} PC, kationische Guar-Derivate, kationische Honig-Derivate, insbesondere das Handelsprodukt Honeyquat^{®} 50, kationische Alkylpolyglycodside gemäß der DE-PS 44 13 686 und Polymere vom Typ Polyquaternium-37.

Weiterhin sind kationisierte Proteinhydrolysate zu den kationischen Polymeren zu zählen, wobei das zugrunde liegende Proteinhydrolysat vom Tier, beispielsweise aus Collagen, Milch oder Keratin, von der Pflanze, beispielsweise aus Weizen, Mais, Reis, Kartoffeln, Soja oder Mandeln, von marinen Lebensformen, beispielsweise aus Fischcollagen oder Algen, oder biotechnologisch gewonnenen Proteinhydrolysaten, stammen kann. Die den erfindungsgemäßen kationischen Derivaten zugrunde liegenden Proteinhydrolysate können aus den entsprechenden Proteinen durch eine chemische, insbesondere alkalische oder saure Hydrolyse, durch eine enzymatische Hydrolyse und/oder einer Kombination aus beiden Hydrolysearten gewonnen werden. Die Hydrolyse von Proteinen ergibt in der Regel ein Proteinhydrolysat mit einer Molekulargewichtsverteilung von etwa 100 Dalton bis hin zu mehreren tausend Dalton. Bevorzugt sind solche kationischen Proteinhydrolysate, deren zugrunde liegender Proteinanteil ein Molekulargewicht von 100 bis zu 25000 Dalton, bevorzugt 250 bis 5000 Dalton aufweist. Weiterhin sind unter kationischen Proteinhydrolysaten quaternierte Aminosäuren und deren Gemische zu verstehen. Die Quaternisierung der Proteinhydrolysate oder der Aminosäuren wird häufig mittels quarternären Ammoniumsalzen wie beispielsweise N,N-Dimethyl-N-(n-Alkyl)-N-(2-hydroxy-3-chloro-n-propyl)-ammoniumhalogeniden durchgeführt. Weiterhin können die kationischen Proteinhydrolysate auch noch weiter derivatisiert sein. Als typische Beispiele für die erfindungsgemäßen kationischen Proteinhydrolysate und -derivate seien die unter den INCI - Bezeichnungen im "International Cosmetic Ingredient Dictionary and Handbook", (seventh edition 1997, The Cosmetic, Toiletry, and Fragrance Association 1101 17^{th} Street, N.W., Suite 300, Washington, DC 20036-4702) genannten und im Handel erhältlichen Produkte genannt: Cocodimonium Hydroxypropyl Hydrolyzed Collagen, Cocodimopnium Hydroxypropyl Hydrolyzed Casein, Cocodimonium Hydroxypropyl Hydrolyzed Collagen, Cocodimonium Hydroxypropyl Hydrolyzed Hair Keratin, Cocodimonium Hydroxypropyl Hydrolyzed Keratin, Cocodimonium Hydroxypropyl Hydrolyzed Rice Protein, Cocodimonium Hydroxypropyl Hydrolyzed Soy Protein, Cocodimonium Hydroxypropyl Hydrolyzed Wheat Protein, Hydroxypropyl Arginine Lauryl/Myristyl Ether HCl, Hydroxypropyltrimonium Gelatin, Hydroxypropyltrimonium Hydrolyzed Casein, Hydroxypropyltrimonium Hydrolyzed Collagen, Hydroxypropyltrimonium Hydrolyzed Conchiolin Protein, Hydroxypropyltrimonium Hydrolyzed Keratin, Hydroxypropyltrimonium Hydrolyzed Rice Bran Protein, Hydroxypropyltrimonium Hydrolyzed Soy Protein, Hydroxypropyl Hydrolyzed Vegetable Protein, Hydroxypropyltrimonium Hydrolyzed Wheat Protein, Hydroxypropyltrimonium Hydrolyzed Wheat Protein/Siloxysilicate, Laurdimonium Hydroxypropyl Hydrolyzed Soy Protein, Laurdimonium Hydroxypropyl Hydrolyzed Wheat Protein, Laurdimonium Hydroxypropyl Hydrolyzed Wheat Protein/Siloxysilicate, Lauryldimonium Hydroxypropyl Hydrolyzed Casein, Lauryldimonium Hydroxypropyl Hydrolyzed Collagen, Lauryldimonium Hydroxypropyl Hydrolyzed Keratin, Lauryldimonium Hydroxypropyl Hydrolyzed Soy Protein, Steardimonium Hydroxypropyl Hydrolyzed Casein, Steardimonium Hydroxypropyl Hydrolyzed Collagen, Steardimonium Hydroxypropyl Hydrolyzed Keratin, Steardimonium Hydroxypropyl Hydrolyzed Rice Protein, Steardimonium Hydroxypropyl Hydrolyzed Soy Protein, Steardimonium Hydroxypropyl Hydrolyzed Vegetable Protein, Steardimonium Hydroxypropyl Hydrolyzed Wheat Protein, Steartrimonium Hydroxyethyl Hydrolyzed Collagen, Quaternium-76 Hydrolyzed Collagen, Quaternium-79 Hydrolyzed Collagen, Quaternium-79 Hydrolyzed Keratin, Quaternium-79 Hydrolyzed Milk Protein, Quaternium-79 Hydrolyzed Soy Protein, Quaternium-79 Hydrolyzed Wheat Protein.

Ganz besonders bevorzugt sind die kationischen Proteinhydrolysate und -derivate auf pflanzlicher Basis.

Bevorzugt eingesetzte amphotere Polymere sind solche Polymerisate, die sich im wesentlichen zusammensetzen aus
(a) Monomeren mit quartären Ammoniumgruppen der allgemeinen Formel (M-I),

   R¹-CH=CR²-CO-Z-(CₙH₂ₙ)-N⁽⁺⁾R³R⁴R⁵ A⁽⁻⁾ (M-I)

   in der R¹ und R² unabhängig voneinander stehen für Wasserstoff oder eine Methylgruppe und R³, R⁴ und R⁵ unabhängig voneinander für Alkylgruppen mit 1 bis 4 Kohlenstoffatomen, Z eine NH-Gruppe oder ein Sauerstoffatom, n eine ganze Zahl von 2 bis 5 und A⁽⁻⁾ das Anion einer organischen oder anorganischen Säure ist, und
(b) monomeren Carbonsäuren der allgemeinen Formel (M-II),

   R⁶-CH=CR⁷-COOH (M-II)

   in denen R⁶ und R⁷ unabhängig voneinander Wasserstoff oder Methylgruppen sind.

Diese Verbindungen können sowohl direkt als auch in Salzform, die durch Neutralisation der Polymerisate, beispielsweise mit einem Alkalihydroxid, erhalten wird, erfindungsgemäß eingesetzt werden. Bezüglich der Einzelheiten der Herstellung dieser Polymerisate wird ausdrücklich auf den Inhalt der deutschen Offenlegungsschrift 39 29 973 Bezug genommen. Ganz besonders bevorzugt sind solche Polymerisate, bei denen Monomere des Typs (a) eingesetzt werden, bei denen R³, R⁴ und R⁵ Methylgruppen sind, Z eine NH-Gruppe und A⁽⁻⁾ ein Halogenid-, Methoxysulfat- oder Ethoxysulfat-Ion ist; Acrylamidopropyl-trimethylammoniumchlorid ist ein besonders bevorzugtes Monomeres (a). Als Monomeres (b) für die genannten Polymerisate wird bevorzugt Acrylsäure verwendet.

Die erfindungsgemäßen farbverändernden Mittel enthalten die kationischen Polymere bevorzugter Weise in einer Menge von 0,01 bis 5 Gew.-%, insbesondere in einer Menge von 0,1 bis 2 Gew.-%, jeweils bezogen auf die gesamte Anwendungszubereitung.

Die zu entfärbenden keratinhaltigen Fasern sind bevorzugt mit Oxidationsfarbstoffen und/oder direktziehenden Farbstoffen, als Vertreter der synthetischen Farbstoffe, gefärbt.

Als zur Färbung der zu entfärbenden keratinhaltigen Fasern verwendeten Entwicklerkomponenten können prinzipiell nachfolgende Entwicklerkomponenten dienen.

Es kann erfindungsgemäß bevorzugt sein, als Entwicklerkomponente ein p-Phenylendiaminderivat oder eines seiner physiologisch verträglichen Salze einzusetzen. Besonders bevorzugt sind p-Phenylendiaminderivate der Formel (E1) 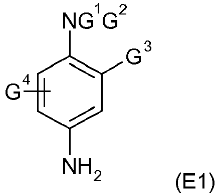 wobei
- G¹: steht für ein Wasserstoffatom, einen (C₁ bis C₄)-Alkylrest, einen (C₁ bis C₄)-Monohydroxyalkylrest, einen (C₂ bis C₄)-Polyhydroxyalkylrest, einen (C₁ bis C₄)-Alkoxy-(C, bis C₄)-alkylrest, einen 4'-Amino'henylrest oder einen (C₁ bis C₄)-Alkylrest, der mit einer stickstoffhaltigen Gruppe, einem Phenyl- oder einem 4'-Amino'henylrest substituiert ist;
- G²: steht für ein Wasserstoffatom, einen (C₁ bis C₄)-Alkylrest, einen (C₁ bis C₄)-Monohydroxyalkylrest, einen (C₂ bis C₄)-Polyhydroxyalkylrest, einen (C₁ bis C₄)-Alkoxy-(C, bis C₄)-alkylrest oder einen (C₁ bis C₄)-Alkylrest, der mit einer stickstoffhaltigen Gruppe substituiert ist;
- G³: steht für ein Wasserstoffatom, ein Halogenatom, wie ein Chlor-, Brom-, lod- oder Fluoratom, einen (C₁ bis C₄)-Alkylrest, einen (C₁ bis C₄)-Monohydroxyalkylrest, einen (C₂ bis C₄)-Polyhydroxyalkylrest, einen (C₁ bis C₄)-Hydroxyalkoxyrest, einen (C₁ bis C₄)-Acetylaminoalkoxyrest, einen Mesylamino-(C₁ bis C₄)-alkoxyrest oder einen (C₁ bis C₄)-Carbamoylaminoalkoxyrest;
- G⁴: steht für ein Wasserstoffatom, ein Halogenatom oder einen (C₁ bis C₄)-Alkylrest oder
wenn G³ und G⁴ in ortho-Stellung zueinander stehen, können sie gemeinsam eine verbrückende α,ω-Alkylendioxogruppe, wie beispielsweise eine Ethylendioxygruppe bilden.

Besonders bevorzugte p-Phenylendiamine der Formel (E1) werden ausgewählt aus einer oder mehrerer Verbindungen der Gruppe, die gebildet wird, aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)-anilin, N,N-Bis-((3-hydroxyethyl)-p-phenylendiamin, 4-N,N-Bis-(β-hydroxyethyl)-amino-2-methylanilin, 4-N,N-Bis-(β-hydroxyethyl)-amino-2-chloranilin, 2-(β-Hydroxyethyl)-p-phenylendiamin, 2-(α,β-Dihydroxyethyl)-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin, N-(β,γ-Dihydroxypropyl)-p-phenylendiamin, N-(4'-Amino'henyl)-p-phenylendiamin, N-Phenyl-p-phenylendiamin, 2-(β-Hydroxyethyloxy)-p-phenylendiamin, 2-(β-Acetylaminoethyloxy)-p-phenylendiamin, N-(β-Methoxyethyl)-p-phenylendiamin, N-(4-Amino-3-methylphenyl)-N-[3-(1H-imidazol-1-yl)propyl]amin, 5,8-Diaminobenzo-1,4-dioxan sowie ihren physiologisch verträglichen Salzen.

Erfindungsgemäß ganz besonders bevorzugte p-Phenylendiaminderivate der Formel (E1) sind ausgewählt aus mindestens einer Verbindung der Gruppe p-Phenylendiamin, p-Toluylendiamin, 2-(β-Hydroxyethyl)-p-phenylendiamin, 2-(α,β-Dihydroxyethyl)-p-phenylendiamin, N,N-Bis-(β-hydroxyethyl)-p-phenylendiamin, N-(4-Amino-3-methylphenyl)-N-[3-(1H-imidazol-1-yl)propyl]amin, sowie den physiologisch verträglichen Salzen dieser Verbindungen.

Es kann erfindungsgemäß weiterhin zur Färbung des Substrats bevorzugt sein, als Entwicklerkomponente Verbindungen einzusetzen, die mindestens zwei aromatische Kerne enthalten, die mit Amino- und/oder Hydroxylgruppen substituiert sind. Unter den zweikernigen Entwicklerkomponenten sind insbesondere die Verbindungen geeignet, die der folgenden Formel (E2) entsprechen, sowie ihre physiologisch verträglichen Salze: 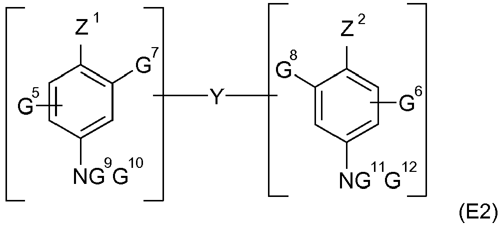 wobei:
- Z¹ und Z²: stehen unabhängig voneinander für einen Hydroxyl- oder NH₂-Rest, der gegebenenfalls durch einen (C₁ bis C₄)-Alkylrest, durch einen (C₁ bis C₄)-Hydroxyalkylrest und/oder durch eine Verbrückung Y substituiert ist oder der gegebenenfalls Teil eines verbrückenden Ringsystems ist,
- die Verbrückung Y: steht für eine Alkylengruppe mit 1 bis 14 Kohlenstoffatomen, wie beispielsweise eine lineare oder verzweigte Alkylenkette oder einen Alkylenring, die von einer oder mehreren stickstoffhaltigen Gruppen und/oder einem oder mehreren Heteroatomen wie Sauerstoff-, Schwefel- oder Stickstoffatomen unterbrochen oder beendet sein kann und eventuell durch einen oder mehrere Hydroxyl- oder (C₁ bis C₈)-Alkoxyreste substituiert sein kann, oder eine direkte Bindung,
- G⁵ und G⁶: stehen unabhängig voneinander für ein Wasserstoff- oder Halogenatom, einen (C₁ bis C₄)-Alkylrest, einen (C₁ bis C₄)-Monohydroxyalkylrest, einen (C₂ bis C₄)-Polyhydroxyalkylrest, einen (C₁ bis C₄)-Aminoalkylrest oder eine direkte Verbindung zur Verbrückung Y,
- G⁷, G⁸, G⁹, G¹⁰, G¹¹ und G¹²: stehen unabhängig voneinander für ein Wasserstoffatom, eine direkte Bindung zur Verbrückung Y oder einen (C₁ bis C₄)-Alkylrest,
mit der Maßgabe, dass die Verbindungen der Formel (E2) nur eine Verbrückung Y pro Molekül enthalten. Die in Formel (E2) verwendeten Substituenten sind erfindungsgemäß analog zu den obigen Ausführungen definiert.

Bevorzugte zweikernige Entwicklerkomponenten der Formel (E2) werden insbesondere aus mindestens einer der folgenden Verbindungen ausgewählt: N,N'-Bis-('-hydroxyethyl)-N,N'-bis-("-amino'henyl)-1,3-diamino-propan-2-ol, N,N'-Bis-('-hydroxyethyl)-N,N'-bis-("-amino'henyl)-ethylendiamin, N,N'-Bis-("-aminophenyl)-tetramethylendiamin, N,N'-Bis-('-hydroxyethyl)-N,N'-bis-("-aminophenyl)-tetramethylendiamin, N,N'-Bis-('-(methylamino)phenyl)-tetramethylendiamin, N,N'-Dieth'I-N,N'-bis-("-amino'3'-methy'phenyl)-ethylendiamin, Bis-(2-hydroxy-5-aminophenyl)-methan, N,N'-Bis-("-amino'henyl)-1,4-diazacycloheptan, N,N'-Bis-('-hydroxy-5-aminobenzyl)-piperazin, N-(4'-Amino'henyl)-p-phenylendiamin und 1,10-Bis-(2',5'-di'mi'ophenyl)-1,4,7,10-tetraoxadecan sowie ihre physiologisch verträglichen Salze. Ganz besonders bevorzugte zweikernige Entwicklerkomponenten der Formel (E2) werden ausgewählt unter N,N'-Bis-('-hydroxyethyl)-N,N'-bis-('-aminophenyl)-1,3-diamino-propan-2-ol, Bis-(2-hydroxy-5-aminophenyl)-methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan oder eines der physiologisch verträglichen Salze dieser Verbindungen.

Weiterhin kann es erfindungsgemäß bevorzugt sein, als Entwicklerkomponente ein p-Aminophenolderivat oder eines seiner physiologisch verträglichen Salze zur Färbung der zu entfärbenden keratinhaltigen Fasern einzusetzen. Besonders bevorzugt sind p-Aminophenolderivate der Formel (E3) 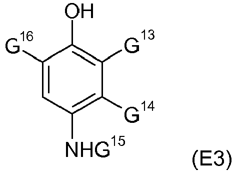 wobei:
- G¹³: steht für ein Wasserstoffatom, ein Halogenatom, einen (C₁ bis C₄)-Alkylrest, einen (C₁ bis C₄)-Monohydroxyalkylrest, einen (C₂ bis C₄)-Polyhydroxyalkylrest, einen (C₁ bis C₄)-Alkoxy-(C₁ bis C₄)-alkylrest, einen (C₁ bis C₄)-Aminoalkylrest, einen Hydroxy-(C₁ bis C₄)-alkylaminorest, einen (C₁ bis C₄)-Hydroxyalkoxyrest, einen (C₁ bis C₄)-Hydroxyalkyl-(C₁ bis C₄)-aminoalkylrest oder einen (Di-[(C₁ bis C₄)-alkyl]amino)-(C₁ bis C₄)-alkylrest, und
- G¹⁴: steht für ein Wasserstoff- oder Halogenatom, einen (C₁ bis C₄)-Alkylrest, einen (C₁ bis C₄)-Monohydroxyalkylrest, einen (C₂ bis C₄)-Polyhydroxyalkylrest, einen (C₁ bis C₄)-Alkoxy-(C₁ bis C₄)-alkylrest, einen (C₁ bis C₄)-Aminoalkylrest oder einen (C₁ bis C₄)-Cyanoalkylrest,
- G¹⁵: steht für Wasserstoff, einen (C₁ bis C₄)-Alkylrest, einen (C₁ bis C₄)-Monohydroxyalkylrest, einen (C₂ bis C₄)-Polyhydroxyalkylrest, einen Phenylrest oder einen Benzylrest, und
- G¹⁶: steht für Wasserstoff oder ein Halogenatom.

Die in Formel (E3) verwendeten Substituenten sind erfindungsgemäß analog zu den obigen Ausführungen definiert.

Bevorzugte p-Aminophenole der Formel (E3) sind insbesondere p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(β-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(β-hydroxyethyl-aminomethyl)-phenol, 4-Amino-2-(α,β-dihydroxyethyl)-phenol, 4-Amino-2-fluorphenol, 4-Amino-2-chlorphenol, 4-Amino-2,6-dichlorphenol, 4-Amino-2-(diethyl-aminomethyl)-phenol sowie ihre physiologisch verträglichen Salze.

Ganz besonders bevorzugte Verbindungen der Formel (E3) sind p-Aminophenol, 4-Amino-3-methylphenol, 4-Amino-2-aminomethylphenol, 4-Amino-2-(α,β-dihydroxyethyl)-phenol und 4-Amino-2-(diethylaminomethyl)-phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin kann die Entwicklerkomponente zur Färbung der zu entfärbenden keratinhaltigen Fasern ausgewählt sein aus heterocyclischen Entwicklerkomponenten, wie beispielsweise aus Pyrimidinderivaten, Pyrazolderivaten, Pyrazolopyrimidin-Derivaten bzw. ihren physiologisch verträglichen Salzen.

Bevorzugte Pyrimidin-Derivate werden erfindungsgemäß ausgewählt aus Verbindungen gemäß Formel (E4) bzw. deren physiologisch verträglichen Salzen, 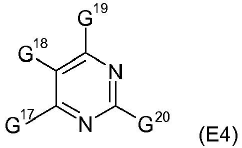 worin
- G¹⁷, G¹⁸ und G¹⁹: unabhängig voneinander für ein Wasserstoffatom, eine Hydroxygruppe, eine (C₁ bis C₄)-Alkoxygruppe oder eine Aminogruppe steht und
- G²⁰: für eine Hydroxygruppe oder eine Gruppe -NG²¹G²- steht, worin G²¹ und G²² unabhängig voneinander stehen für ein Wasserstoffatom, eine (C₁ bis C₄)-Alkylgruppe, eine (C₁ bis C₄)-Monohydroxyalkylgruppe,
mit der Maßgabe, dass maximal zwei der Gruppen aus G¹⁷, G¹⁸, G¹⁹ und G²⁰ eine Hydroxygruppe bedeuten und höchstens zwei der Reste G¹⁷, G¹⁸ und G¹⁹für ein Wasserstoffatom stehen. Dabei ist es wiederum bevorzugt, wenn gemäß Formel (E4) mindestens zwei Gruppen aus G¹⁷, G¹⁸, G¹⁹ und G²⁰ für eine Gruppe -NG²¹G²- stehen und höchstens zwei Gruppen aus G¹⁷, G¹⁸, G¹⁹ und G²⁰ für eine Hydroxygruppe stehen.

Besonders bevorzugte Pyrimidin-Derivate sind insbesondere die Verbindungen 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin.

Bevorzugte Pyrazol-Derivate werden erfindungsgemäß ausgewählt aus Verbindungen gemäß Formel (E5), 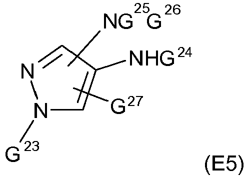 worin
- G²³, G²⁴, G²⁵: stehen unabhängig voneinander für ein Wasserstoffatom, eine (C₁ bis C₄)-Alkylgruppe, eine (C₁ bis C₄)-Monohydroxyalkylgruppe, eine (C₂ bis C₄)-Polyhydroxyalkylgruppe, eine gegebenenfalls substituierte Arylgruppe oder eine gegebenenfalls substituierte Aryl-(C₁ bis C₄)-alkylgruppe, mit der Maßgabe dass, wenn G²⁵ für ein Wasserstoffatom steht, G²⁶ neben den vorgenannten Gruppen zusätzlich für eine Gruppe -NH₂ stehen kann,
- G²⁶: steht für ein Wasserstoffatom, eine (C₁ bis C₄)-Alkylgruppe, eine (C₁ bis C₄)-Monohydroxyalkylgruppe oder eine (C₂ bis C₄)-Polyhydroxyalkylgruppe und
- G²⁷: steht für ein Wasserstoffatom, eine gegebenenfalls substituierte Arylgruppe, eine (C₁ bis C₄)-Alkylgruppe oder eine (C₁ bis C₄)-Monohydroxyalkylgruppe, insbesondere für ein Wasserstoffatom oder eine Methylgruppe.

Bevorzugt bindet in Formel (E5) der Rest -NG²⁵G²- an die 5 Position und der Rest G²⁷ an die 3 Position des Pyrazolzyklus. Besonders bevorzugte Pyrazol-Derivate sind insbesondere die Verbindungen, die ausgewählt werden unter 4,5-Diamino-1-methylpyrazol, 4,5-Diamino-1-(β-hydroxyethyl)-pyrazol, 3,4-Diaminopyrazol, 4,5-Diamino-1-(4'-chlor'enzyl)-pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(β-hydroxyethyl)-3-methylpyrazol, 4,5-Diamino-1-ethyl-3-methylpyrazol, 4,5-Diamino-1-ethyl-3-(4'-metho'yphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(β-aminoethyl)amino-1,3-dimethylpyrazol, sowie deren physiologisch verträglichen Salze.

Ganz besonders bevorzugte Entwicklerkomponenten zur Färbung der zu entfärbenden keratinhaltigen Fasern werden ausgewählt, aus mindestens einer Verbindung aus der Gruppe, die gebildet wird aus p-Phenylendiamin, p-Toluylendiamin, 2-(β-Hydroxyethyl)-p-phenylendiamin, 2-(a,β-Dihydroxyethyl)-p-phenylendiamin, N,N-Bis-(β-hydroxyethyl)-p-phenylendiamin, N-(4-Amino-3-methylphenyl)-N-[3-(1H-imidazol-1-yl)propyl]amin, N,N'-Bis-('-hydroxyethyl)-N,N'-bis-('-aminophenyl)-1,3-diamino-propan-2-ol, Bis-(2-hydroxy-5-aminophenyl)-methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(α,β-dihydroxyethyl)-phenol und 4-Amino-2-(diethylaminomethyl)-phenol, 4,5-Diamino-1-(β-hydroxyethyl)-pyrazol, 2,4,5,6-Tetraamino-pyrimidin, 4-Hydroxy-2,5,6-triaminopyrimidin, 2-Hydroxy-4,5,6-triaminopyrimidin, sowie den physiologisch verträglichen Salzen dieser Verbindungen.

Im folgenden werden Beispiele für die als Substituenten der Verbindungen der Formeln (E1) bis (E5) genannten Reste aufgezählt: Beispiele für (C₁ bis C₄)-Alkylreste sind die Gruppen -CH₃, -CH₂CH₃, -CH₂CH₂CH₃, -CH(CH₃)₂, -CH₂CH₂CH₂CH₃, -CH₂CH(CH₃)₂, -CH(CH₃)CH₂CH₃, -C(CH₃)₃. Erfindungsgemäße Beispiele für (C₁ bis C₄)-Alkoxyreste sind -OCH₃, -OCH₂CH₃, -OCH₂CH₂CH₃, -OCH(CH₃)₂, -OCH₂CH₂CH₂CH₃, -OCH₂CH(CH₃)₂, -OCH(CH₃)CH₂CH₃, -OC(CH₃)₃, insbesondere eine Methoxy- oder eine Ethoxygruppe.

Weiterhin können als bevorzugte Beispiele für eine (C₁ bis C₄)-Monohydroxyalkylgruppe-CH₂OH,-
-CH₂CH₂OH, -CH₂CH₂CH₂OH, -CHCH(OH)CH₃, -CH₂CH₂CH₂CH₂OH, wobei die Gruppe -CH₂CH₂OH bevorzugt ist.

Ein besonders bevorzugtes Beispiel einer (C₂ bis C₄)-Polyhydroxyalkylgruppe ist die 1,2-Dihydroxyethylgruppe.

Beispiele für Halogenatome sind F-, Cl- oder Br-Atome, Cl-Atome sind ganz besonders bevorzugte Beispiele.

Beispiele für stickstoffhaltige Gruppen sind insbesondere -NH₂, (-₁ bis C₄)-Monoalkylaminogruppen, (C₁ bis C₄)-Dialkylaminogruppen, (C₁ bis C₄)-Trialkylammoniumgruppen, (C₁ bis C₄)-Monohydroxyalkylaminogruppen, Imidazolinium und - NH₃⁺.

Beispiele für (C₁ bis C₄)-Monoalkylaminogruppen sind -NHCH₃,--NHCH₂CH₃, -NHCH₂CH₂CH₃, -NHCH(CH₃)₂.

Beispiele für (C₁ bis C₄)-Dialkylaminogruppe sind -N(CH₃)₋, -N(CH₂CH₃)₂.

Beispiele für (C₁ bis C₄)-Trialkylammoniumgruppen sind -N⁺(CH₃-₃, -N⁺(CH₃)₂(CH₂CH₃), -N⁺(CH₃)(CH₂CH₃)₂.

Beispiele für (C₁ bis C₄)-Hydroxyalkylaminoreste sind -NH-CH₂-H₂OH, -NH-CH₂CH₂OH, -NH-CH₂CH₂CH₂OH, -NH-CH₂CH₂CH₂OH.

Beispiele für (C₁ bis C₄)-Alkoxy-(C₁ bis C₄)-alkylgruppen sind die Gruppen -CH₂CH₂-O-CH₃, -CH₂CH₂CH₂-O-CH₃, -CH₂CH₂-O-CH₂CH₃, -CH₂CH₂CH₂-O-CH₂CH₃, -CH₂CH₂-O-CH(CH₃), -CH₂CH₂CH₂-O-CH(CH₃).

Beispiele für Hydroxy-(C₁ bis C₄)-alkoxyreste sind -O-CH₂O-, -O-CH₂CH₂OH -O-CH₂CH₂CH₂OH, -O-CHCH(OH)CH₃, -O-CH₂CH₂CH₂CH₂OH.

Beispiele für (C₁ bis C₄)-Acetylaminoalkoxyreste sind -O-CH₂N-C(O)CH₃, -O-CH₂CH₂NHC(O)CH₃,
-O-CH₂CH₂CH₂NHC(O)CH₃, -O-CH₂CH(NHC(O)CH₃)CH₃, -O-CH₂CH₂CH₂CH₂NHC(O)CH₃.

Beispiele für (C₁ bis C₄)-Carbamoylaminoalkoxyreste sind -O-CH₂C-₂-NH-C(O)-NH₂, -O-CH₂CH₂CH₂-NH-C(O)-NH₂, -O-CH₂CH₂CH₂CH₂-NH-C(O)-NH₂.

Beispiele für (C₁ bis C₄)-Aminoalkylreste sind -CH₂NH₂- -CH₂CH₂NH₂, -CH₂CH₂CH₂NH₂, -CH₂CH(NH₂)CH₃, -CH₂CH₂CH₂CH₂NH₂.

Beispiele für (C₁ bis C₄)-Cyanoalkylreste sind -CH₂CN,--CH₂CH₂CN, -CH₂CH₂CH₂CN.

Beispiele für (C₁ bis C₄)-Hydroxyalkylamino-(C₁ bis C₄)-alkylreste sind -CH₂CH₂-H-CH₂CH₂OH, -CH₂CH₂CH₂NH-CH₂CH₂OH, -CH₂CH₂NH-CH₂CH₂CH₂OH, -CH₂CH₂CH₂NH-CH₂CH₂CH₂OH.

Beispiele für Di[(C₁ bis C₄)-Hydroxyalkyl]amino-(C₁ bis C₄)-alkylreste sind -CH₂CH₂N(CH₂CH₂OH)₂, -CH₂CH₂CH₂N(CH₂CH₂OH)₂, -CH₂CH₂N(CH₂CH₂CH₂OH)₂, -CH₂CH₂CH₂N(CH₂CH₂CH₂OH)₂.

Ein Beispiel für Arylgruppen ist die Phenylgruppe.

Beispiele für Aryl-(C₁ bis C₄)-alkylgruppen sind die Benzylgruppe und die 2-Phenylethylgruppe.

Kupplerkomponenten bilden im Rahmen der oxidativen Färbung allein keine signifikante Färbung aus, sondern benötigen stets die Gegenwart von Entwicklerkomponenten. Daher ist es erfindungsgemäß bevorzugt, dass zur Färbung der zu entfärbenden keratinhaltigen Fasern bei Verwendung mindestens einer Entwicklerkomponente zusätzlich mindestens eine Kupplerkomponente zum Einsatz kommt.

Kupplerkomponenten im Sinne der Erfindung erlauben mindestens eine Substitution eines chemischen Restes des Kupplers durch die oxidierte Form der Entwicklerkomponente. Dabei bildet sich eine kovalente Bindung zwischen Kuppler- und Entwicklerkomponente aus. Kuppler sind bevorzugt zyklische Verbindungen, die am Zyklus mindestens zwei Gruppen tragen, ausgewählt aus (i) gegebenenfalls substituierten Aminogruppen und/oder (ii) Hydroxygruppen. Wenn die zyklische Verbindung ein Sechsring (bevorzugt aromatisch) ist, so befinden sich die besagten Gruppen bevorzugt in ortho-Position oder meta-Position zueinander. Erfindungsgemäße Kupplerkomponenten zur Färbung der zu entfärbenden keratinhaltigen Fasern werden bevorzugt als mindestens eine Verbindung aus einer der folgenden Klassen ausgewählt:
- m-Aminophenol und/oder dessen Derivate,
- m-Diaminobenzol und/oder dessen Derivate,
- o-Diaminobenzol und/oder dessen Derivate,
- o-Aminophenolderivate, wie beispielsweise o-Aminophenol,
- Naphthalinderivate mit mindestens einer Hydroxygruppe,
- Di- beziehungsweise Trihydroxybenzol und/oder deren Derivate,
- Pyridinderivate,
- Pyrimidinderivate,
- Monohydroxyindol-Derivate und/oder Monoaminoindol-Derivate,
- Monohydroxyindolin-Derivate und/oder Monoaminoindolin-Derivate,
- Pyrazolonderivate, wie beispielsweise 1-Phenyl-3-methylpyrazol-5-on,
- Morpholinderivate wie beispielsweise 6-Hydroxybenzomorpholin oder 6-Amino-benzomorpholin,
- Chinoxalinderivate wie beispielsweise 6-Methyl-1,2,3,4-tetrahydrochinoxalin,

Gemische aus zwei oder mehrer Verbindungen aus einer oder mehrerer dieser Klassen sind im Rahmen dieser Ausführungsform ebenso erfindungsgemäß.

Die erfindungsgemäß verwendbaren m-Aminophenole bzw. deren Derivate werden bevorzugt ausgewählt aus mindestens einer Verbindung der Formel (K1) und/oder aus mindestens einem physiologisch verträglichen Salz einer Verbindung gemäß Formel (K1), 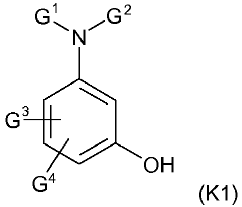 worin
- G¹ und G²: unabhängig voneinander stehen für ein Wasserstoffatom, eine (C₁ bis C₄)-Alkylgruppe, eine (C₃ bis C₆)-Cycloalkylgruppe, eine (C₂ bis C₄)-Alkenylgruppe, eine (C₁ bis C₄)-Monohydroxyalkylgruppe, eine (C₂ bis C₄)-Polyhydroxyalkylgruppe, eine (C₂ bis C₄)-Perfluoracylgruppe, eine Aryl-(C₁ bis C₆)-alkylgruppe, eine Amino-(C₁ bis C₆)-alkylgruppe, eine (C₁ bis C₆)-Dialkylamino-(C₁ bis C₆)-alkylgruppe oder eine (C₁ bis C₆)-Alkoxy-(C, bis C₆)-alkylgruppe, wobei G¹ und G² gemeinsam mit dem Stickstoffatom einen fünfgliedrigen, sechsgliedrigen oder siebengliedrigen Ring bilden können,
- G³ und G⁴: unabhängig voneinander stehen für ein Wasserstoffatom, ein Halogenatom, eine (C₁ bis C₄)-Alkylgruppe, eine (C₁ bis C₄)-Alkoxygruppe, eine Hydroxygruppe, eine (C₁ bis C₄)-Monohydroxyalkylgruppe, eine (C₂ bis C₄)-Polyhydroxyalkylgruppe, eine Hydroxy-(C₁ bis C₄)-alkoxygruppe, eine (C₁ bis C₆)-Alkyox-(C₂ bis C₆)-alkoxygruppe, eine Arylgruppe oder eine Heteroarylgruppe.

Besonders bevorzugte m-Aminophenol-Kupplerkomponenten werden ausgewählt aus mindestens einer Verbindung aus der Gruppe, die gebildet wird aus m-Aminophenol, 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydro'yethyl)-amino-2-methylphenol, 3-(Diethylamino)-phenol, N-Cyclopentyl-3-aminophenol, 1,3-Dihydroxy-5-(methylamino)-benzol, 3-Ethylamino-4-methylphenol, 2,4-Dichlor-3-aminophenol und den physiologisch verträglichen Salzen aller vorstehend genannten Verbindungen.

Die erfindungsgemäß verwendbaren m-Diaminobenzole bzw. deren Derivate werden bevorzugt ausgewählt aus mindestens einer Verbindung der Formel (K2) und/oder aus mindestens einem physiologisch verträglichen Salz einer Verbindung gemäß Formel (K2), 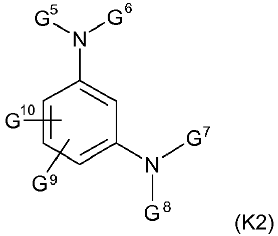 worin
- G⁵, G⁶, G⁷ und G⁸: unabhängig voneinander stehen für ein Wasserstoffatom, eine (C₁ bis C₄)-Alkylgruppe, eine (C₃ bis C₆)-Cycloalkylgruppe, eine (C₂ bis C₄)-Alkenylgruppe, eine (C₁ bis C₄)-Monohydroxyalkylgruppe, eine (C₂ bis C₄)-Polyhydroxyalkylgruppe, eine (C₁ bis C₄)-Alkoxy-(C₁ bis C₄)-alkylgruppe, eine Aryl-(C₁ bis C₄)-alkylgruppe, eine Heteroaryl-(C₁ bis C₄)-alkylgruppe, eine (C₂ bis C₄)-Perfluoracylgruppe, oder gemeinsam mit dem Stickstoffatom einen fünfgliedrigen oder sechsgliedrigen Heterozyklus bilden
- G⁹ und G¹⁰: unabhängig voneinander stehen für ein Wasserstoffatom, ein Halogenatom, eine (C₁ bis C₄)-Alkylgruppe, eine ω-(2,4-Diaminophenyl)-(C₁ bis C₄)-alkylgruppe, eine ω-(2,4-Diaminophenyloxy)-(C₁ bis C₄)-alkoxygruppe, eine (C₁ bis C₄)-Alkoxygruppe, eine Hydroxygruppe, eine (C₁ bis C₄)-Alkoxy-(C₂ bis C₄)-alkoxygruppe, eine Arylgruppe, eine Heteroarylgruppe, eine (C₁ bis C₄)-Monohydroxyalkylgruppe, eine (C₂ bis C₄)-Polyhydroxyalkylgruppe, eine Hydroxy-(C₁ bis C₄)-alkoxygruppe.

Besonders bevorzugte m-Diaminobenzol-Kupplerkomponenten werden ausgewählt aus mindestens einer Verbindung aus der Gruppe, die gebildet wird aus m-Phenylendiamin, 2-(2,4-Diaminophenoxy)ethanol, 1,3-Bis(2,4-diaminophenoxy)propan, 1-Methoxy-2-amino-4-(2'-hydro'yethylamino)benzol, 1,3-Bis(2,4-diaminophenyl)propan, 2,6-Bis(2'-hydro'yethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2'-hydro'yethyl)-aminobenzol und den physiologisch verträglichen Salzen aller vorstehend genannten Verbindungen.

Die erfindungsgemäß verwendbaren o-Diaminobenzole bzw. deren Derivate werden bevorzugt ausgewählt aus mindestens einer Verbindung der Formel (K3) und/oder aus mindestens einem physiologisch verträglichen Salz einer Verbindung gemäß Formel (K3), 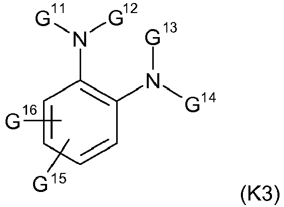 worin
- G¹¹, G¹², G¹³ und G¹⁴: unabhängig voneinander stehen für ein Wasserstoffatom, eine (C₁ bis C₄)-Alkylgruppe, eine (C₃ bis C₆)-Cycloalkylgruppe, eine (C₂ bis C₄)-Alkenylgruppe, eine (C₁ bis C₄)-Monohydroxyalkylgruppe, eine (C₂ bis C₄)-Polyhydroxyalkylgruppe, eine (C₁ bis C₄)-Alkoxy-(C, bis C₄)-alkylgruppe, eine Aryl-(C₁ bis C₄)-alkylgruppe, eine Heteroaryl-(C₁ bis C₄)-alkylgruppe, eine (C₂ bis C₄)-Perfluoracylgruppe, oder gemeinsam mit dem Stickstoffatom einen fünfgliedrigen oder sechsgliedrigen Heterozyklus bilden
- G¹⁵ und G¹⁶: unabhängig voneinander stehen für ein Wasserstoffatom, ein Halogenatom, eine Carboxylgruppe, eine (C₁ bis C₄)-Alkylgruppe, eine (C₁ bis C₄)-Alkoxygruppe, eine Hydroxygruppe, eine (C₁ bis C₄)-Monohydroxyalkylgruppe, eine (C₂ bis C₄)-Polyhydroxyalkylgruppe, eine Hydroxy-(C₁ bis C₄)-alkoxygruppe.

Besonders bevorzugte o-Diaminobenzol-Kupplerkomponenten werden ausgewählt aus mindestens einer Verbindung aus der Gruppe, die gebildet wird aus 3,4-Diaminobenzoesäure und 2,3-Diamino-1-methylbenzol und den physiologisch verträglichen Salzen aller vorstehend genannten Verbindungen.

Bevorzugte Di- beziehungsweise Trihydroxybenzole und deren Derivate werden ausgewählt aus mindestens einer Verbindung der Gruppe, die gebildet wird aus Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol.

Die erfindungsgemäß verwendbaren Pyridinderivate werden bevorzugt ausgewählt aus mindestens einer Verbindung der Formel (K4) und/oder aus mindestens einem physiologisch verträglichen Salz einer Verbindung gemäß Formel (K4), 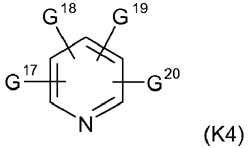 worin
- G¹⁷ und G¹⁸: stehen unabhängig voneinander für eine Hydroxygruppe oder eine Gruppe - NG²¹G²⁻,
worin G²¹ und G²² unabhängig voneinander stehen für ein Wasserstoffatom, eine (C₁ bis C₄)-Alkylgruppe, eine (C₃ bis C₆)-Cycloalkylgruppe, eine (C₂ bis C₄)-Alkenylgruppe, eine Arylgruppe, eine (C₁ bis C₄)-Monohydroxyalkylgruppe, eine (C₂ bis C₄)-Polyhydroxyalkylgruppe, eine (C₁ bis C₄)-Alkoxy-(C₁ bis C₄)-alkylgruppe, eine Aryl-(C₁ bis C₄)-alkylgruppe, eine Heteroaryl-(C₁ bis C₄)-alkylgruppe,
- G¹⁹ und G²⁰: stehen unabhängig voneinander für ein Wasserstoffatom, ein Halogenatom, eine (C₁ bis C₄)-Alkylgruppe oder eine (C₁ bis C₄)-Alkoxygruppe.

Es ist bevorzugt, wenn gemäß Formel (K4) die Reste G¹⁷ und G¹⁸ in ortho-Position oder in meta-Position zueinander stehen.

Besonders bevorzugte Pyridinderivate werden ausgewählt aus mindestens einer Verbindung der Gruppe, die gebildet wird aus 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin, 3,5-Diamino-2,6-dimethoxypyridin, 3,4-Diaminopyridin, 2-(2-Methoxyethyl)amino-3-amino-6-methoxypyridin, 2-(4'-Methoxyphenyl)amino-3-aminopyridin, und den physiologisch verträglichen Salzen der vorgenannten Verbindungen.

Bevorzugte Naphthalinderivate mit mindestens einer Hydroxygruppe werden ausgewählt aus mindestens einer Verbindung der Gruppe, die gebildet wird aus 1-Naphthol, 2-Methyl-1-naphthol, 2-Hydroxymethyl-1-naphthol, 2-Hydroxyethyl-1-naphthol, 1,3-Dihydroxynaphthalin, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin.

Die erfindungsgemäß verwendbaren Indolderivate werden bevorzugt ausgewählt aus mindestens einer Verbindung der Formel (K5) und/oder aus mindestens einem physiologisch verträglichen Salz einer Verbindung gemäß Formel (K5), 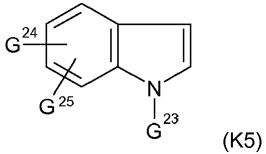 worin
- G²³: steht für ein Wasserstoffatom, eine (C₁ bis C₄)-Alkylgruppe, eine (C₃ bis C₆)-Cycloalkylgruppe, eine (C₂ bis C₄)-Alkenylgruppe, eine (C₁ bis C₄)-Monohydroxyalkylgruppe, eine (C₂ bis C₄)-Polyhydroxyalkylgruppe, eine Aryl-(C₁ bis C₄)-alkylgruppe,
- G²⁴: steht für eine Hydroxygruppe oder eine Gruppe -NG²⁶G²⁷, worin G²⁶ und G²⁷ unabhängig voneinander stehen für ein Wasserstoffatom, eine (C₁ bis C₄)-Alkylgruppe, eine (C₃ bis C₆)-Cycloalkylgruppe, eine (C₂ bis C₄)-Alkenylgruppe, eine (C₁ bis C₄)-Monohydroxyalkylgruppe, eine (C₂ bis C₄)-Polyhydroxyalkylgruppe,
- G²⁵: Wasserstoffatom, ein Halogenatom oder eine (C₁ bis C₄)-Alkylgruppe,

mit der Maßgabe, dass G²⁴ in meta-Position oder ortho-Position zum Strukturfragment NG²³ der Formel bindet.

Besonders bevorzugte Indolderivate werden ausgewählt aus mindestens einer Verbindung der Gruppe, die gebildet wird aus 4-Hydroxyindol, 6-Hydroxyindol und 7-Hydroxyindol und den physiologisch verträglichen Salzen der vorgenannten Verbindungen.

Die erfindungsgemäß verwendbaren Indolinderivate werden bevorzugt ausgewählt aus mindestens einer Verbindung der Formel (K6) und/oder aus mindestens einem physiologisch verträglichen Salz einer Verbindung gemäß Formel (K6), 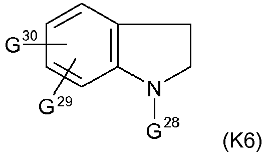 worin
- G²⁸: steht für ein Wasserstoffatom, eine (C₁ bis C₄)-Alkylgruppe, eine (C₃ bis C₆)-Cycloalkylgruppe, eine (C₂ bis C₄)-Alkenylgruppe, eine (C₁ bis C₄)-Monohydroxyalkylgruppe, eine (C₂ bis C₄)-Polyhydroxyalkylgruppe, eine Aryl-(C₁ bis C₄)-alkylgruppe,
- G²⁹: steht für eine Hydroxygruppe oder eine Gruppe -NG³¹G³², worin G³¹ und G³² unabhängig voneinander stehen für ein Wasserstoffatom, eine (C₁ bis C₄)-Alkylgruppe, eine (C₃ bis C₆)-Cycloalkylgruppe, eine (C₂ bis C₄)-Alkenylgruppe, eine (C₁ bis C₄)-Monohydroxyalkylgruppe, eine (C₂ bis C₄)-Polyhydroxyalkylgruppe,
- G³⁰: Wasserstoffatom, ein Halogenatom oder eine (C₁ bis C₄)-Alkylgruppe,
mit der Maßgabe, dass G²⁹ in meta-Position oder ortho-Position zum Strukturfragment NG²⁸ der Formel bindet.

Besonders bevorzugte Indolinderivate werden ausgewählt aus mindestens einer Verbindung der Gruppe, die gebildet wird aus 4-Hydroxyindolin, 6-Hydroxyindolin und 7-Hydroxyindolin und den physiologisch verträglichen Salzen der vorgenannten Verbindungen.

Bevorzugte Pyrimidinderivate werden ausgewählt aus mindestens einer Verbindung der Gruppe, die gebildet wird aus 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin und den physiologisch verträglichen Salzen der vorgenannten Verbindungen.

Zur Färbung der zu entfärbenden keratinhaltigen Fasern werden erfindungsgemäß besonders bevorzugte Kupplerkomponenten ausgewählt unter m-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2'-Hydro'yethyl)-amino-2-methylphenol, 2,4-Dichlor-3-aminophenol, o-Aminophenol, m-Phenylendiamin, 2-(2,4-Diaminophenoxy)ethanol, 1,3-Bis(2,4-diaminophenoxy)propan, 1-Methoxy-2-amino-4-(2'-hydro'yethylamino)benzol, 1,3-Bis(2,4-diaminophenyl)propan, 2,6-Bis(2'-hydro'yethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}-amino)ethanol, 2-[3-Morpholin-4-ylphenyl)amino]ethanol, 3-Amino-4-(2-methoxyethoxy)-5-methylphenylamin, 1-Amino-3-bis-(2'-hydro'yethyl)-aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder den physiologisch verträglichen Salzen der vorgenannten Verbindungen.

Die Kupplerkomponenten werden bevorzugt in einer Menge von 0,005 bis 20 Gew.-%, vorzugsweise 0,1 bis 5 Gew.-%, jeweils bezogen auf das zur Färbung der zu entfärbenden keratinhaltigen Fasern angewendete Oxidationsfärbemittel, verwendet.

Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1:0,5 bis 1:3, insbesondere 1:1 bis 1:2, stehen können.

Im folgenden werden Beispiele für die als Substituenten der Verbindungen der Formeln (K1) bis (K6) genannten Reste aufgezählt: Beispiele für (C₁ bis C₄)-Alkylreste sind die Gruppen -CH₃, -CH₂CH₃, -CH₂CH₂CH₃, -CH(CH₃)₂, -CH₂CH₂CH₂CH₃, -CH₂CH(CH₃)₂, -CH(CH₃)CH₂CH₃, -C(CH₃)₃.

Erfindungsgemäße Beispiele für (C₃ bis C₆)-Cycloalkylgruppen sind die Cyclopropyl, die Cyclopentyl und die Cyclohexylgruppe.

Erfindungsgemäße Beispiele für (C₁ bis C₄)-Alkoxyreste sind -OCH₃, -OCH₂CH₃, -OCH₂CH₂CH₃, -OCH(CH₃)₂, -OCH₂CH₂CH₂CH₃, -OCH₂CH(CH₃)₂, -OCH(CH₃)CH₂CH₃, -OC(CH₃)₃, insbesondere eine Methoxy- oder eine Ethoxygruppe.

Weiterhin können als bevorzugte Beispiele für eine (C₁ bis C₄)-Monohydroxyalkylgruppe-CH₂OH,-CH₂CH₂OH, -CH₂CH₂CH₂OH, -CH₂CH(OH)CH₃, -CH₂CH₂CH₂CH₂OH genannt werden, wobei die Gruppe -CH₂CH₂-H bevorzugt ist.

Ein besonders bevorzugtes Beispiel einer (C₂ bis C₄)-Polyhydroxyalkylgruppe ist die 1,2-Dihydroxyethylgruppe.

Beispiele für Halogenatome sind F-, Cl- oder Br-Atome, Cl-Atome sind ganz besonders bevorzugte Beispiele.

Beispiele für stickstoffhaltige Gruppen sind insbesondere -NH₂, (-₁ bis C₄)-Monoalkylaminogruppen, (C₁ bis C₄)-Dialkylaminogruppen, (C₁ bis C₄)-Trialkylammoniumgruppen, (C₁ bis C₄)-Monohydroxyalkylaminogruppen, Imidazolinium und - NH₃⁺.

Beispiele für (C₁ bis C₄)-Monoalkylaminogruppen sind -NHCH₃,--NHCH₂CH₃, -NHCH₂CH₂CH₃, -NHCH(CH₃)₂.

Beispiele für (C₁ bis C₄)-Dialkylaminogruppe sind -N(CH₃)₋, -N(CH₂CH₃)₂.

Beispiele für (C₁ bis C₄)-Alkoxy-(C₁ bis C₄)-alkylgruppen sind die Gruppen -CH₂CH₂-O-CH₃, -CH₂CH₂CH₂-O-CH₃, -CH₂CH₂-O-CH₂CH₃, -CH₂CH₂CH₂-O-CH₂CH₃, -CH₂CH₂-O-CH(CH₃)₂, -CH₂CH₂CH₂-O-CH(CH₃)₂.

Beispiele für (C₁ bis C₄)-Alkoxy-(C₁ bis C₄)-alkoxygruppen sind die Gruppen -O-CH₂C-₂-O-CH₃, -O-CH₂CH₂CH₂-O-CH₃, -O-CH₂CH₂-O-CH₂CH₃, -O-CH₂CH₂CH₂-O-CH₂CH₃, -O-CH₂CH₂-O-CH(CH₃)₂, -O-CH₂CH₂CH₂-O-CH(CH₃)₂.

Beispiele für Hydroxy-(C₁ bis C₄)-alkoxyreste sind -O-CH₂O-, -O-CH₂CH₂OH, -O-CH₂CH₂CH₂OH,
-O-CH₂CH(OH)CH₃, -O-CH₂CH₂CH₂CH₂OH.

Beispiele für (C₁ bis C₄)-Aminoalkylreste sind -CH₂NH₂- -CH₂CH₂NH₂, -CH₂CH₂CH₂NH₂, -CH₂CH(NH₂)CH₃, -CH₂CH₂CH₂CH₂NH₂.

Ein Beispiel für Arylgruppen ist die Phenylgruppe, die auch substituiert sein kann.

Beispiele für Aryl-(C₁ bis C₄)-alkylgruppen sind die Benzylgruppe und die 2-Phenylethylgruppe. Weiterhin können die zu entfärbenden keratinhaltigen Fasern auch mit in der Natur vorkommenden, natürlichen Farbstoffen, wie sie beispielsweise in Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten sind, gefärbt sein.

Die zu entfärbenden keratinhaltigen Fasern können entweder mit direktziehenden Farbstoffe allein oder in Kombination von direktziehenden Farbstoffen mit Oxidationsfarbstoffen gefärbt worden sein. Bevorzugt geeignete direktziehende Farbstoffe sind Nitrophenylendiamine, Nitroaminophenole, Azofarbstoffe, Anthrachinone oder Indophenole. Bevorzugt zur Entfärbung geeignete direktziehende Farbstoffe sind die unter den internationalen Bezeichnungen bzw. Handelsnamen HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)-amino-2-nitrobenzol, 3-Nitro-4-(β-hydroxyethyl)-aminophenol, 2-(2'-Hydro'yethyl)amino-4,6-dinitrophenol, 1-(2'-Hydro'yethyl)amino-4-methyl-2-nitrobenzol, 1-Amine-4-(2'-hydro'yethyl)-amino-5-chlor-2-nitrobenzol, 4-Amino-3-nitrophenol, 1-(2'-Ureid'ethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol. Bevorzugte erfindungsgemäße Mittel sind dadurch gekennzeichnet, daß sie zusätzlich mindestens einen direktziehenden Farbstoff, vorzugsweise in einer Menge von 0,01 bis 20 Gew.-%, bezogen auf das gesamte Färbemittel, enthalten.

Ein zweiter Gegenstand der Erfindung ist die Verwendung eines Mittels gemäß erstem Erfindungsgegenstand zur Entfärbung keratinhaltiger Fasern, insbesondere menschlicher Haare. Ein dritter Gegenstand der Erfindung ist ein Verfahren zur reduktiven Entfärbung von keratinhaltigen Fasern, insbesondere menschlichen Haaren, in dem ein Mittel des ersten Erfindungsgegenstandes auf die keratinhaltigen Fasern aufgetragen wird und nach einer Einwirkzeit wieder abgespült wird.

Die keratinhaltigen Fasern wurden bevorzugt mit Oxidationsfarbstoffen und/oder direktziehenden Farbstoffen eingefärbt.

Die Einwirkzeit beträgt bevorzugt 1 bis 60 Minuten, bevorzugt 5 bis 30 Minuten. Die Einwirkung des erfindungsgemäßen Mittels kann nicht nur bei Raumtemperatur, sondern bevorzugt in einem Temperaturbereich von 15 bis 60 °C, insbesondere von 25 bis 60 °C erfolgen.

In einer bevorzugten Ausführungsform des Verfahrens wird unmittelbar vor dem Auftragen des erfindungsgemäßen Mittels des ersten Erfindungsgegenstandes das anwendungsbereite Mittel durch mischen einer Zusammensetzung, enthaltend, gegebenenfalls in einem kosmetischen Träger, mindestens eine organische Verbindung der Formel (I) mit einer Zusammensetzung, enthaltend in einem kosmetischen Träger, mindestens eine organische Verbindung, ausgewählt aus der Gruppe, die gebildet wird, aus
(i) cyclischen, organischen Carbonaten und
   C₄-C₁₂-Fettsäuredimethylamiden
hergestellt.

Dabei können besagte Zusammensetzungen vor der Vermischung wie weiter unten beschrieben konfektioniert bzw. beschaffen sein.

Nach Ablauf der Einwirkzeit werden die keratinhaltigen Fasern ausgespült, wobei bevorzugt ein tensidhaltiges Mittel, wie beispielsweise ein Reinigungsmittel oder ein Shampoo, Anwendung findet. Gegebenenfalls kann das Substrat mehrfach ausgespült, bzw. mit dem tensidhaltigen Mittel behandelt werden.

Nach dem Ausspülen kann es vorteilhaft sein, die keratinhaltigen Fasern mit einer Oxidationsmittelhaltigen Zusammensetzung zu behandeln. Bevorzugt wird Wasserstoffperoxid als Oxidationsmittel, bevorzugt in Konzentrationen von 0,5 bis 6 Gew.-%, eingesetzt. Die Einwirkzeit beträgt bevorzugt 1 bis 30 Minuten, besonders bevorzugt 1 bis 10 Minuten. Nach Ablauf der Einwirkzeit wird die Oxidationsmittelhaltige Zusammensetzug ausgespült.

Es ist erfindungsgemäß bevorzugt das erfindungsgemäße Mittel als Mehrkomponentensystem in Form eines Kit-of-parts bereitzustellen. Dabei ist es bevorzugt, dass in einem ersten Kontainer eine Zusammensetzung, enthaltend, gegebenenfalls in einem kosmetischen Träger, mindestens eine organische Verbindung der Formel (I) und in einem davon getrennten zweiten Kontainer eine Zusammensetzung, enthaltend in einem kosmetischen Träger,
- mindestens eine organische Verbindung, ausgewählt aus der Gruppe, die gebildet wird, aus
   (i) cyclischen, organischen Carbonaten und
      C₄-C₁₂-Fettsäuredimethylamiden,
- mindestens einen (C₁₀ bis C₃₀)-Fettalkohol und
- mindestens einen Emulgator ausgewählt aus der Gruppe, umfassend
   mit mindestens einem (C₁₀ bis C₃₀)- Kohlenwasserstoffrest substituierte amphotere Emulgatoren
konfektioniert ist.

Als Kontainer verstehen sich erfindungsgemäß Behältnisse wie beispielsweise Flaschen, Sachets, Beutel, Tuben, Dosen und viele andere mehr. Auch eine Kammer eines Mehrkammerbehältnisses gilt im Sinne der Erfindung als Kontainer. In einem Mehrkammerbehältnis werden die jeweiligen Zusammensetzungen in unterschiedlichen Kammern konfektioniert und werden unmittelbar vor dem Austritt aus dem Mehrkammerbehältnis oder danach zusammengebracht und dadurch gemischt.

Die Zusammensetzung des ersten Kontainers liegt bevorzugt als Festkörper, insbesondere pulverförmig, granuliert oder als Formkörper, vor.

Eine pulverförmige Zusammensetzung des ersten Kontainers besitzt eine bevorzugte mittlere Teilchengröße von 0,0001 bis 100 µm, insbesondere von 0,005 bis 10 µm. Diese Pulver können durch Beschichtung mit Fetten, Ölen oder Wachsen, wie beispielsweise Silikonölen, flüssigen Kohlenwasserstoffen, Dialkylethern, Fettsäuren, Fettalkoholen, entstaubt werden.

Als Granulate werden erfindungsgemäß körnige Partikel verstanden. Diese körnigen Partikel sind fließfähig.

Granulate können durch Feuchtgranulierung, durch Trockengranulierung bzw. Kompaktierung und durch Schmelzerstarrungsgranulierung hergestellt werden. Die gebräuchlichste Granuliertechnik ist die Feuchtgranulierung, da diese Technik den wenigsten Einschränkungen unterworfen ist und am sichersten zu Granulaten mit günstigen Eigenschaften führt. Die Feuchtgranulierung erfolgt durch Befeuchtung der Pulvermischungen mit Lösungsmitteln und/oder Lösungsmittelgemischen und/oder Lösungen von Bindemitteln und/oder Lösungen von Klebstoffen und wird vorzugsweise in Mischern, Wirbelbetten oder Sprühtürmen durchgeführt, wobei besagte Mischer beispielsweise mit Rühr- und Knetwerkzeugen ausgestattet sein können. Für die Granulation sind jedoch auch Kombinationen von Wirbelbett(en) und Mischer(n), bzw. Kombinationen verschiedener Mischer einsetzbar. Die Granulation erfolgt unter Einwirkung niedriger bis hoher Scherkräfte.

Wenn die Zusammensetzung des ersten Kontainers als Formkörper vorliegt, dann können diese erfindungsgemäßen Formkörper jedwede geometrische Form aufweisen, wie beispielsweise konkave, konvexe, bikonkave, bikonvexe, kubische, tetragonale, orthorhombische, zylindrische, sphärische, zylindersegmentartige, scheibenförmige, tetrahedrale, dodecahedrale, octahedrale, konische, pyramidale, ellipsoide, fünf-, sieben- und achteckig-prismatische sowie rhomboedrische Formen. Auch völlig irreguläre Grundflächen wie Pfeil- oder Tierformen, Bäume, Wolken usw. können realisiert werden. Die Ausbildung als Tafel, die Stab- bzw. Barrenform, Würfel, Quader und entsprechende Raumelemente mit ebenen Seitenflächen sowie insbesondere zylinderförmige Ausgestaltungen mit kreisförmigem oder ovalem Querschnitt und Formkörper mit sphärischer Geometrie sind erfindungsgemäß bevorzugt. Besonders bevorzugt sind Formkörper in Gestalt sphärischer Geometrie.

Die zylinderförmige Ausgestaltung erfaßt die Darbietungsform von der Tablette bis zu kompakten Zylinderstücken mit einem Verhältnis von Höhe zu Durchmesser größer 1. Weist der Basisformkörper Ecken und Kanten auf, so sind diese vorzugsweise abgerundet. Als zusätzliche optische Differenzierung ist eine Ausführungsform mit abgerundeten Ecken und abgeschrägten ("angefasten") Kanten bevorzugt.

Die sphärische Ausgestaltung umfaßt eine kugelförmigen Gestalt auch einen Hybrid aus Kugel- und Zylinderform, wobei jede Grundfläche des Zylinders mit je einer Halbkugel überkappt ist. Die Halbkugeln haben bevorzugt einen Radius von ca. 4 mm und der gesamte Formkörper dieser Ausgestaltung eine Länge von 12 - 14 mm.

Ein erfindungsgemäßer Formkörper mit sphärischer Ausgestaltung kann nach den bekannten Verfahren hergestellt werden. Es ist dabei möglich, die Formkörper durch Extrusion eines Vorgemisches mit nachfolgender Formgebung zu produzieren, wie es zum Beispiel in der WO-A-91/02047 näher ausgeführt ist, auf die im Rahmen dieser Anmeldung ausdrücklich Bezug genommen wird.

In einer weiteren bevorzugten Ausführungsform werden daher nahezu kugelförmige Formkörper, insbesondere durch Extrusion und nachfolgender Verrundung zur Formgebung, hergestellt.

In einer weiteren Ausführungsform können die portionierten Preßlinge dabei jeweils als voneinander getrennte Einzelelemente ausgebildet sein, die der vorbestimmten Dosiermenge der besagten Thiolgruppen-haltigen Verbindung entspricht. Ebenso ist es aber möglich, Preßlinge auszubilden, die eine Mehrzahl solcher Masseneinheiten in einem Preßling verbinden, wobei insbesondere durch vorgegebene Sollbruchstellen die leichte Abtrennbarkeit portionierter kleinerer Einheiten vorgesehen ist. Die Ausbildung der portionierten Preßlinge als Tabletten in Zylinder- oder Quaderform kann zweckmäßig sein, wobei ein Durchmesser/Höhe-Verhältnis im Bereich von etwa 0,5 : 2 bis 2 : 0,5 bevorzugt ist. Handelsübliche Hydraulikpressen, Exzenterpressen oder Rundläuferpressen sind geeignete Vorrichtungen insbesondere zur Herstellung derartiger Preßlinge.

Eine weitere mögliche Raumform der erfindungsgemäßen Formkörper weist eine rechteckige Grundfläche auf, wobei die Höhe der Formkörper kleiner ist als die kleinere Rechteckseite der Grundfläche. Abgerundete Ecken sind bei dieser Angebotsform bevorzugt.

Unabhängig von seiner Erscheinungsform als Pulver, Granulat bzw. Formkörper enthält Die Zusammensetzung des ersten Kontainers bevorzugt mindestens einen der nachfolgenden Zusätze:
Die Zusammensetzung des ersten Kontainers enthält bevorzugt zusätzlich mindestens einen Auflösungsbeschleuniger. Dies ist insbesondere dann bevorzugt, wenn Die Zusammensetzung des ersten Kontainers granuliert oder als Formkörper vorliegt. Der Begriff Auflösungsbeschleuniger umfasst dabei Gas-entwickelnde Komponenten, vorgebildete und eingeschlossene Gase, Sprengmittel sowie deren Mischungen.

Die beschleunigte Auflösung der Zusammensetzung des ersten Kontainers kann erfindungsgemäß auch durch Vorgranulierung der weiteren Bestandteile erreicht werden.

In einer bevorzugten Ausführungsform der erfindungsgemäßen Zusammensetzungen des ersten Kontainers enthalten diese zur Auflösungsbeschleunigung, insbesondere neben mindestens einem Sprengmittel auf Cellulosebasis, ein Gemisch aus Stärke und mindestens einem Saccharid. Disaccharide sind bevorzugt verwendete Saccharide dieser Ausführungsform. Das besagte Gemisch liegt bevorzugt in einem Gewichtsverhältnis von Stärke und den eingesetzten Sacchariden von 10 : 1 bis 1 : 10, besonders bevorzugt von 1 : 1 bis 1 : 10, ganz besonders bevorzugt von 1 : 4 bis 1 : 8 in der Zusammensetzung des ersten Kontainers vor.

Die verwendeten Disaccharide sind bevorzugt ausgewählt aus Lactose, Maltose, Saccharose, Trehalose, Turanose, Gentiobiose, Melibiose und Cellobiose. Besonders bevorzugt werden Lactose, Maltose und Saccharose und ganz besonders bevorzugt Lactose in den erfindungsgemäßen Formkörpern eingesetzt.

Die Stärke-Saccharid-Mischung ist in der Zusammensetzung des ersten Kontainers in einer Menge von 5 bis 70 Gew.%, bevorzugt von 20 bis 40 Gew.% bezogen auf die Masse des gesamten Mittels A, enthalten.

Die Zusammensetzung des zweiten Kontainers umfasst bevorzugt einem bei Anwendungsbedingungen flüssigen kosmetischen Träger. Dies gilt insbesondere dann, wenn die Zusammensetzung des ersten Kontainers pulverförmig, granuliert oder als Formkörper vorliegt.

Das Kit kann zusätzlich Applikationshilfen, wie beispielsweise Pinsel oder Mascarabürste, enthalten.

Das Kit kann zusätzlich Schutzhandschuhe enthalten.

Das Kit kann weiterhin zusätzlich einen Konditioner und/oder ein Shampoo enthalten.

Für die Erfindungsgegenstände zwei bis drei gelten *mutatis mutandis* die Definitionen und Ausführungsformen, die im Rahmen des ersten Erfindungsgegenstandes Erwähnung finden.

Der Erfindungsgegenstand soll exemplarisch anhand folgender Beispiele erläutert werden.

### Beispiele

### 1. Herstellung der Rezeptur

Folgende Rezeptur wurde wie nachfolgend beschrieben bereitgestellt.

Die Mengenangaben sind Gewichtsprozent bezogen auf das Gewicht des jeweiligen Mittels.

| Rohstoff | Gew.-% |
|---|---|
| L- | 4,00 |
| Propylencarbonat | 20,00 |
| Ajidew^{®} NL 50 | 1,00 |
| Cetearylalkohol | 5,00 |
| Lorol^{®}, techn. | 1,00 |
| Eumulgin^{®} B2 | 0,50 |
| Dehyton^{®} PK 45 | 3,00 |
| Euxyl^{®} PE 9010 | 0,70 |
| Zitronensäuremonohydrat | 0,50 |
| Wasser | ad 100 |

- Ajidew^{®} NL50: Natriumsalz der Pyrrolidoncarbonsäure (ca. 48-52% Aktivsubstanzgehalt; INCI-Bezeichnung: Sodium PCA) (Ajinomoto)
- Dehyton^{®}PK45: N,N-Dimethyl-N-(kokosamidopropyl)ammoniumaceto-betain (ca. 38% Aktivsubstanz; INCI-Bezeichnung: Aqua (Water), Cocamidopropyl Betaine) (Cognis)
- Eumulgin^{®} B2: Cetylstearylalkohol mit ca. 20 EO-Einheiten (INCI-Bezeichnung: Ceteareth-20) (Cognis)
- Lorol^{®} tech.: C₁₂₋₁₈-Fettalkohol (INCI-Bezeichnung: Coconut alcohol) (Cognis)
- Euxyl^{®} PE 9010: Mischung aus 90 Gew.-% 2-Phenoxyethanol und 10 Gew.-% 3-(2-Ethylhexyloxy)-1,2-propandiol (100 Gew.-% Aktivsubstanz, INCI-Bezeichnung: Phenoxyethanol, Ethylhexyl Glycerin) (Schülke & Mayr)

Die angegebene Wassermenge wurde in einem ausreichend großen und hitzebeständigen Gefäß vorgelegt und auf 80°C erwärmt. Unter Rühren wurden nacheinander die folgenden Rohstoffe zugegeben: Cetearylalkohol, Lorol techn, Eumulgin B2, Dehyton PK 45

Diese Rohstoffe wurden ungeschmolzen dem Ansatz hinzugefügt und 30 Minuten bei 80°C gerührt, bis eine homogene Emulsion entstand. Anschließend wurde der Ansatz unter gleichzeitigem Rühren auf 35 - 40°C abgekühlt. Danach wurden unter Rühren folgende Rohstoffe zugegeben: Ajidew NL-50, Euxyl PE 9010, Zitronensäuremonohydrat, Propylencarbonat

Es wurde für weitere 30 Minuten gerührt bis eine homogene Emulsion/Mischung entstand. Der Ansatz wurde sodann in geeignete Lagergefäße überführt.

Die Rezeptur wurde für 6 Monate bei einer Temperatur von 40°C gelagert. Die Viskosität blieb über den Lagerzeitraum erhalten.

Vor Anwendung des Farbabzugsmittels wurde separat konfektioniertes L-Cysteinhydrochloridmonohydrat zugegeben und gerührt, bis sich das Salz löste. Die Viskosität blieb erhalten.

Mit Oxidationshaarfarbe gefärbte Haare ließen sich mit der Anwendungsmischung hervorragend entfärben.

## Patentansprüche

1. Mittel zur reduktiven Entfärbung keratinhaltiger Fasern, insbesondere menschlicher Haare, mit einem pH-Wert von 2 bis 6,5 enthaltend in einem Träger eine Wirkstoffkombination aus
(a) mindestens einer organischen Verbindung der Formel (I)
HS-X-COOM _{(I)}
worin
X ein gesättigtes oder ungesättigtes, lineares oder verzweigtes sowie aliphatisches Kohlenwasserstoffgerüst bedeutet, das gegebenenfalls mit mindestens einer der folgenden Gruppen substituiert ist
- Thiolgruppe
- Carboxylgruppe
- Carboxylatgruppe
- Hydroxygruppe
- -NH₂
- (C, bis C₆)-Alkylamino
- (C₁ bis C₆)-Dialkylamino
- (C₁ bis C₆)-Hydroxyalkyl
M steht für ein Wasserstoffatom, eine (C₁ bis C₈)-Alkylgruppe oder ein Äquivalent eines ein- oder mehrwertigen Kations, und
(b) mindestens einer organischen Verbindung, ausgewählt aus
(i) cyclischen, organischen Carbonaten und
(ii) C₄-C₁₂-Fettsäuredimethylamiden,
(c) mindestens einen (C₁₀ bis C₃₀)-Fettalkohol und
(d) mindestens einen Emulgator ausgewählt aus mit mindestens einem (C₁₀ bis C₃₀)-Kohlenwasserstoffrest substituierten amphoteren Emulgatoren.

2. Mittel nach Anspruch 1, **dadurch gekennzeichnet, daß** Komponente (a) ausgewählt wird aus mindestens einem Vertreter der Gruppe, die gebildet wird, aus L-Cystein (Säure oder Salz), D-Cystein (Säure oder Salz), D,L-Cystein (Säure oder Salz) und Acetylcystein.

3. Mittel nach einem der Ansprüche 1 bis 2, **dadurch gekennzeichnet, daß** die Komponente (b)(i) ausgewählt wird, aus mindestens einem cyclischer Kohlensäureester der Formel (II-1) 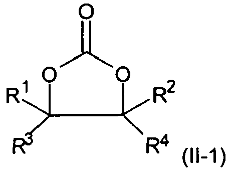 worin die Reste R¹, R², R³ und R⁴ unabhängig voneinander für ein Wasserstoffatom oder organische Reste, insbesondere Alkyl-, Alkenyl- oder Alkylaryl, stehen, die zusätzlich mit weiteren Gruppen, insbesondere Hydroxygruppen, substituiert sein können.

4. Mittel nach einem der Ansprüche 1 bis 3, **dadurch gekennzeichnet, daß** die Komponente (b)(ii) ausgewählt wird, aus mindestens einer Verbindung der Formel (IV) 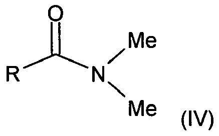 worin
R eine lineare oder verzweigte (C₄ bis C₁₂)-Alkylgruppe bedeutet.

5. Mittel nach einem der Ansprüche 1 bis 4, **dadurch gekennzeichnet, daß** es die Verbindungen der Komponente (a) in einer Menge von 1 bis 10 Gew.-%, insbesondere von 1 bis 5 Gew.-%, jeweils bezogen auf das Gewicht des gesamten Mittels, enthält.

6. Mittel nach einem der Ansprüche 1 bis 5, **dadurch gekennzeichnet, daß** es die Verbindungen der Komponente (b) in einer Menge von 5 Gew.-% bis 50 Gew.-%, insbesondere von 10 Gew.% bis 30 Gew.-%, jeweils bezogen auf das Gewicht des Mittels, enthält.

7. Mittel nach einem der Ansprüche 1 bis 6, **dadurch gekennzeichnet, dass** die (C₁₀ bis C₃₀)-Fettalkohole in einer Menge von 3 bis 10 Gew.-%, besonders bevorzugt von 4 bis 6 Gew.-%, jeweils bezogen auf das Gewicht des erfindungsgemäßen Mittels, enthalten sind.

8. Mittel nach einem der Ansprüche 1 bis 7, **dadurch gekennzeichnet, daß** es einen pH-Wert von pH 2,5 bis pH 5, insbesondere von pH 3 bis pH 4, besitzt.

9. Mittel nach einem der Ansprüche 1 bis 8, **dadurch gekennzeichnet, dass** es eine Viskosität von 2000 bis 10000 mPa·s, insbesondere von 3000 bis 6000 mPa·s aufweist (jeweils Brookfield DV-II+, Spindel 4, 20 UpM bei 20°C).

10. Verwendung eines Mittels gemäß einem der Ansprüche 1 bis 9 zur Entfärbung keratinhaltiger Fasern, insbesondere menschlicher Haare.

11. Verfahren zur reduktiven Entfärbung von keratinhaltigen Fasern, insbesondere menschlichen Haaren, in dem ein Mittel gemäß einem der Ansprüche 1 bis 9 auf die keratinhaltigen Fasern aufgetragen wird und nach einer Einwirkzeit wieder abgespült wird.

## Claims

1. An agent for the reductive decolorization of keratin-containing fibers, in particular human hair, having a pH value from 2 to 6.5 containing, in a carrier, an active agent combination made of
(a) at least one organic compound of formula (I)
HS-X-COOM (I)
where
X denotes a saturated or unsaturated, linear or branched and aliphatic hydrocarbon skeleton, which is optionally substituted with at least one of the following groups:
- thiol group
- carboxyl group
- carboxylate group
- hydroxy group
- -NH₂
- (C₁ to C₆)-alkylamino
- (C₁ to C₆)-dialkylamino
- (C₁ to C₆)-hydroxyalkyl
M denotes a hydrogen atom, a (C₁ to C₈) alkyl group or an equivalent of a .monovalent or polyvalent cation, and
(b) at least one organic compound, selected from
(i) cyclic, organic carbonates and
(ii) i) C₄ to C₁₂ fatty acid dimethylamides,
(c) at least one (C₁₀ to C₃₀) fatty alcohol and
(d) at least one emulsifier selected from amphoteric emulsifiers substituted with at least one (C₁₀ to C₃₀) hydrocarbon group.

2. The agent according to claim 1, **characterized in that** component (a) is selected from at least one representative from the group formed of L-cysteine (acid or salt), D-cysteine (acid or salt), D, L-cysteine (acid or salt), and acetylcysteine.

3. The agent according to either claim 1 or 2, **characterized in that** component (b)(i) is selected from at least one cyclic carbonate ester of formula (II-1) 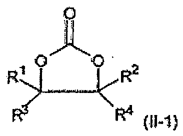 where the groups R¹, R², R³ and R⁴ independently of each other denote a hydrogen atom or organic groups, in particular alkyl, alkenyl or alkyl aryl, which can additionally be substituted with further groups, in particular hydroxy groups.

4. The agent according to one of claims 1 to 3, **characterized in that** component (b)(ii) is selected from at least one compound of formula (IV) 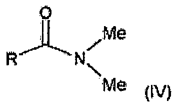 where
R denotes a linear or branched (C₄ to C₁₂) alkyl group.

5. The agent according to one of claims 1 to 4, **characterized by** comprising the compounds of component (a) in a quantity from 1 to 10 wt.%, in particular 1 to 5 wt.%, in each case based on the weight of the total agent.

6. The agent according to one of claims 1 to 5, **characterized by** comprising the compounds of component (b) in a quantity from 5 wt.% to 50 wt.%, in particular 10 wt.% to 30 wt.%, in each case based on the weight of the agent.

7. The agent according to one of claims 1 to 6, **characterized in that** the (C₁₀ to C₃₀) fatty alcohols are present in a quantity from 3 to 10 wt.%, particularly preferably from 4 to 6 wt.%, in each case based on the weight of the agent according to the invention.

8. The agent according to one of claims 1 to 7, **characterized by** having a pH value from pH 2.5 to pH 5, in particular from pH 3 to pH 4.

9. The agent according to one of claims 1 to 8, **characterized by** having a viscosity from 2000 to 10000 mPa·s, in particular from 3000 to 6000 mPa·s (in each case Brookfield DV-II+, spindle 4, 20 rpm at 20°C)

10. Use of an agent according to one of claims 1 to 9 for decolorizing keratin-containing fibers, in particular human hair.

11. A method for the reductive decolorization of keratin-containing fibers, in particular human hair, in which an agent according to one of claims 1 to 9 is applied to the keratin-containing fibers and is rinsed off again after an exposure time.

## Revendications

1. Composition pour la décoloration réductrice de fibres de kératine, en particulier de cheveux humains, dont le pH est dans la gamme de 2 à 6,5 et qui contient un support une combinaison de substances actives constituée de
(a) au moins un composé organique de la formule (I)
HS-X-COOM (I)
dans laquelle
X représente un squelette hydrocarboné saturé ou insaturé, linéaire ou ramifié, et aliphatique qui est éventuellement substitué par au moins un des groupes suivants
- le groupe des thiols
- le groupe des carboxyles
- le groupe des carboxylates
- le groupe hydroxy
- NH₂
- alkylamino en C₁ à C₆,
- dialkylamino en Ci à C₆,
- hydroxyalkyle en Ci à C₆,
M représente un atome d'hydrogène, un groupe alkyle en Ci à C₈ ou un équivalent d'un cation monovalent ou polyvalent, et
(b) au moins un composé organique choisi parmi
(i) les carbonates organiques cycliques, et
(ii) les diéthylamides d'acides gras en C₄ à C₁₂,
(c) au moins un alcool gras en C₁₀ à C₃₀, et
(d) au moins un émulsifiant choisi parmi au moins les émulsifiants amphotères substitués avec un radical hydrocarboné en C₁₀ à C₃₀.

2. Composition selon la revendication 1, **caractérisée en ce que** le composé (a) est choisi parmi au moins un représentant du groupe qui est formé par la L-cystéine (acide ou sel), la D-cystéine (acide ou sel), la D,L-cystéine (acide ou sel) et l'acétylcystéine.

3. Composition selon l'une des revendications 1 à 2, **caractérisée en ce que** le composé (b) (i) est choisi parmi au moins un ester d'acide carbonique cyclique de la formule (11-1) 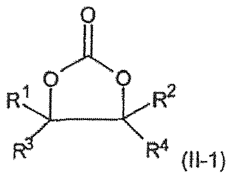 dans laquelle les radicaux R¹, R², R³ et R⁴ sont indépendamment un atome d'hydrogène ou des radicaux organiques, en particulier alkyle, alcényle ou alkylaryle, qui peuvent en outre être substitué par d'autres groupes, notamment des groupes hydroxy.

4. Composition selon l'une des revendications 1 à 3, **caractérisée en ce que** le composé (b) (ii) est choisi parmi au moins un composé de la formule (IV) 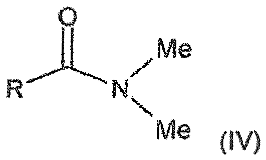 dans laquelle
R représente un groupe alkyle en C₄ à C₁₂ linéaire ou ramifié.

5. Composition selon l'une des revendications 1 à 4, **caractérisée en ce qu'**elle contient les constituants du composés (a) dans une quantité allant de 1 à 10% en poids, en particulier de 1 à 5% en poids, à chaque fois sur la base du poids de la composition totale.

6. Composition selon l'une des revendications 1 à 5, **caractérisée en ce qu'**elle contient les constituants du composé (b) dans une quantité allant de 5% en poids à 50% en poids, en particulier de 10% en poids à 30% en poids, à chaque fois sur la base du poids de la composition.

7. Composition selon l'une des revendications 1 à 6, **caractérisée en ce que** les alcools gras en C₁₀ à C₃₀ sont contenus dans une quantité allant de 3 à 10% en poids, plus préférablement de 4 à 6% en poids, à chaque fois sur la base du poids de la composition de l'invention.

8. Composition selon l'une des revendications 1 à 7, **caractérisée en ce qu'**elle a un pH allant de 2,5 à 5, en particulier de 3 à 4.

9. Composition selon l'une des revendications 1 à 8, **caractérisée en ce qu'**elle a une viscosité de 2000 à 10000 mPas, en particulier de 3000 à 6000 mPas (mesurée à chaque fois avec un Brookfield DV-II+, broche 4, 20 tours par minute à 20°C).

10. Utilisation d'une composition selon l'une des revendications 1 à 9, pour décolorer des fibres de kératine, en particulier des cheveux humains.

11. Procédé de décoloration réductrice de fibres de kératine, en particulier de cheveux humains, dans lequel une composition selon l'une des revendications 1 à 9 est appliquée sur les fibres de kératine et rincée à nouveau après avoir laissé agir.
